# EUROPEAN PATENT APPLICATION

(11) **EP 4 840 861 A1**
(43) Date of publication of application: **18.11.2026**
(21) Application number: 25738384.4
(22) Date of filing: 07.01.2025
(51) Int. Cl.: C07D 491/22, C07D 491/14, A61K 31/4745, A61K 31/496, A61K 47/68, A61P 35/00

(54) **PREPARATION AND USE OF 7-FLUOROALKYL SUBSTITUTED CAMPTOTHECIN DERIVATIVE**

(30) Priority: 08.01.2024 CN 202410028741
(71) Applicant: Hangzhou Adcoris Biopharma Co., Ltd, Hangzhou, Zhejiang 310052 (CN)
(72) Inventor: MIAO, Zhenwei, Hangzhou, Zhejiang 310052 (CN); HUANG, Yunsheng, Hangzhou, Zhejiang 310052 (CN)
(74) Representative: Elzaburu S.L.P.
(86) International application number: PCT/CN2025/070907
(87) International publication number: WO 2025/148853

(57) **Abstract**

The present invention relates to the field of medicines, and in particular to a 7-fluoroalkyl substituted camptothecin derivative compound, and a pharmaceutically acceptable salt thereof, a stereoisomer thereof, a preparation method therefor, and a use thereof in the field of anti-tumor. The camptothecin derivative compound is used as an anti-tumor drug, and has the advantages of high activity, low toxicity, stable metabolism.

## Description

The present application claims priority to Chinese Patent Application 2024100287414 filed on January 08, 2024, which is incorporated herein by reference in its entirety.

### TECHNICAL FIELD

The present disclosure relates to the field of medicine, particularly to the preparation and use of a series of 7-fluoroalkyl-substituted camptothecin derivatives.

### BACKGROUND

Camptothecin and derivatives thereof exhibit inhibitory activity against topoisomerase Top1, especially strong inhibitory activity against a Top1-DNA complex. Camptothecin has remarkable therapeutic efficacy against gastric cancer, esophageal cancer, lung cancer, bladder cancer, etc., and therefore is a broad-spectrum antitumor agent. Camptothecin derivatives irinotecan and topotecan have been approved in many countries for the treatment of various cancers. Another camptothecin derivative Belotecan has been approved in South Korea for the treatment of small cell lung cancer (SCLC) and ovarian cancer. The development of novel camptothecin-based drugs is necessary and boasts a broad application value.

### SUMMARY

An objective of the present disclosure is to provide a 7-fluoroalkyl-substituted aminomethylcamptothecin derivative. As an antitumor agent, the 7-fluoroalkyl-substituted aminomethylcamptothecin derivative has the advantages of high activity, low toxicity, metabolic stability and the like.

In one aspect, the present disclosure provides a compound of Formula (I), and a pharmaceutically acceptable salt and a stereoisomer thereof:

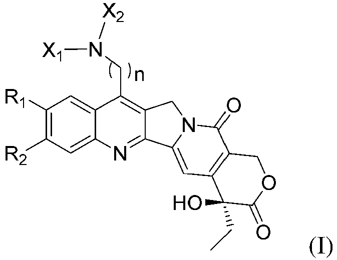

In the formula,
R₁ and R₂ are each independently selected from hydrogen, fluorine, and methyl, or
R₁ and R₂ together with the carbon atoms to which they are attached form 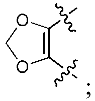
X₁ is selected from hydrogen and C₁-C₃ alkyl;
X₂ is selected from substituted or unsubstituted C₁-C₈ alkyl and substituted or unsubstituted C₃-C₆ cycloalkyl, wherein the C₁-C₈ alkyl or C₃-C₆ cycloalkyl comprises at least one fluorine atom substituent;
n is selected from 1, 2, or 3;
the "substituted" means that one, two, three, or four hydrogen atoms in a group are each independently replaced by a substituent selected from the group consisting of deuterium, halogen, halogen-substituted or unsubstituted C₁₋₆ alkyl, halogen-substituted or unsubstituted C₃₋₆ cycloalkyl, deuterium-substituted or unsubstituted C₁₋₆ alkyl, and deuterium-substituted or unsubstituted C₃₋₆ cycloalkyl.

In an embodiment, X₁ is selected from hydrogen, methyl, ethyl, propyl, and isopropyl.

In an embodiment, X₁ is hydrogen.

In an embodiment, X₂ is selected from C₁-C₈ alkyl substituted with 1, 2, or 3 fluorine, and is further selected from C₁-C₆ alkyl substituted with 1, 2, or 3 fluorine.

In an embodiment, X₂ is selected from substituted or unsubstituted C₁-C₆ alkyl and substituted or unsubstituted C₃-C₆ cycloalkyl, wherein substituted C₁-C₆ alkyl or substituted C₃-C₆ cycloalkyl comprises 1, 2, or 3 fluorine atom substituents.

In an embodiment, X₂ is selected from substituted or unsubstituted C₁-C₆ alkyl, wherein the C₁-C₆ alkyl comprises 1, 2, or 3 fluorine atom substituents.

In an embodiment, X₂ is selected from substituted or unsubstituted C₃-C₆ cycloalkyl, wherein the C₃-C₆ cycloalkyl comprises 1, 2, or 3 fluorine atom substituents.

In an embodiment, X₂ is selected from ethyl, and the ethyl comprises 1, 2, or 3 fluorine atom substituents and is further substituted with 1 or 2 methyl. Such fluorine-substituted ethylamine can significantly reduce basicity of the substituted amine, lower the hepatotoxicity of the compound *in vivo* and improve the metabolic stability and cell membrane permeability, thereby enhancing antitumor activity.

In an embodiment, X₂ is selected from -CH₂-CF₃, -CH(CH₃)-CF₃, or -C(CH₃)₂-CF₃.

In an embodiment, X₂ is selected from

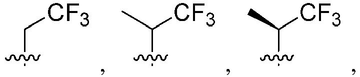

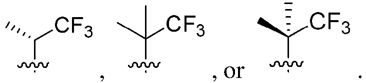

In an embodiment, R₁ is selected from hydrogen, fluorine, and methyl and R₂ is selected from hydrogen and fluorine; or
R₁ and R₂ together with the carbon atoms to which they are attached form

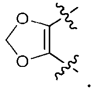

In an embodiment, R₁ is hydrogen; R₂ is hydrogen; or
R₁ is fluorine; R₂ is fluorine; or
R₁ is methyl; R₂ is fluorine; or
R₁ and R₂ together with the carbon atoms to which they are attached form

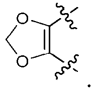

In another aspect, the present disclosure provides a compound of Formula (II), and a pharmaceutically acceptable salt and a stereoisomer thereof:

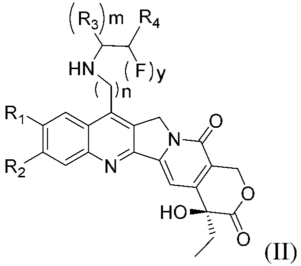

In the formula, R₁, R₂, and n are each the same as defined for the compound of Formula (I).

Further, in Formula (II), R₁ and R₂ are each independently selected from hydrogen, fluorine, and methyl, or
R₁ and R₂ together with the carbon atoms to which they are attached form 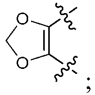
R₃ and R₄ are each independently selected from hydrogen, fluorine, and substituted or unsubstituted C₁-C₆ alkyl, or
R₃ and R₄ together with the carbon atoms to which they are attached form C₃-C₆ cycloalkyl;
n is selected from 1, 2, or 3, preferably 1 or 2;
m is selected from 1 or 2;
y is selected from 1 or 2, preferably 2; and
the "substituted" means that one, two, three, or four hydrogen atoms in a group are each independently replaced by a substituent selected from the group consisting of deuterium, halogen, halogen-substituted or unsubstituted C₁₋₆ alkyl, halogen-substituted or unsubstituted C₃₋₆ cycloalkyl, deuterium-substituted or unsubstituted C₁₋₆ alkyl, and deuterium-substituted or unsubstituted C₃₋₆ cycloalkyl.

In another aspect, the present disclosure provides a compound of Formula (III), and a pharmaceutically acceptable salt and a stereoisomer thereof:

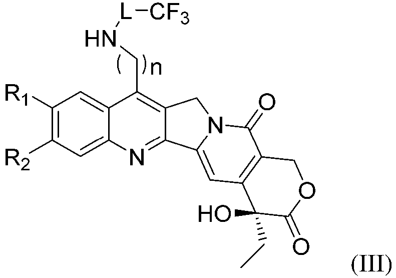

In the formula, R₁, R₂, and n are each the same as defined for the compound of Formula (I);
L is selected from substituted or unsubstituted C₁-C₆ alkyl;
the "substituted" means that one, two, three, or four hydrogen atoms in a group are each independently replaced by a substituent selected from the group consisting of deuterium, halogen, and halogen-substituted or unsubstituted C₁₋₆ alkyl.

In an embodiment, L is selected from -CH₂-, -CH(CH₃)-, or -CH(CH₃)₂-.

In an embodiment, R₁ is selected from hydrogen, fluorine, and methyl; and R₂ is selected from hydrogen and fluorine; or
R₁ and R₂ together with the carbon atoms to which they are attached form

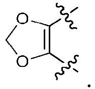

In an embodiment, R₁ is hydrogen; R₂ is hydrogen; or
R₁ is fluorine; R₂ is fluorine; or
R₁ is methyl; R₂ is fluorine; or
R₁ and R₂ together with the carbon atoms to which they are attached form

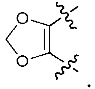

In an embodiment, the compound is selected from compounds represented by Formula (II-1), Formula (II-2), Formula (II-3), or Formula (II-4):

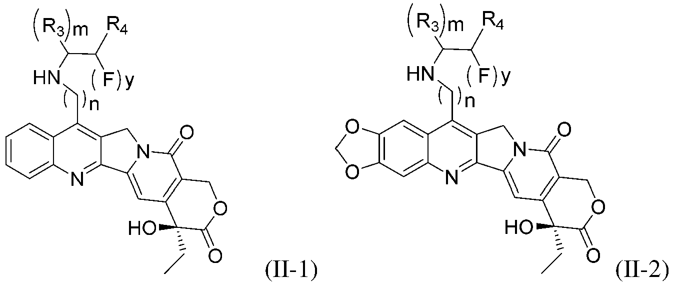

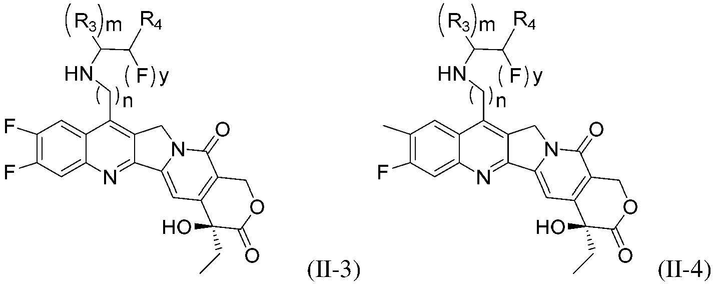

In the formulae, R₃, R₄, n, m, and y are each the same as defined for the compound of Formula (II).

In an embodiment, R₃ and R₄ are each independently selected from hydrogen, fluorine, and substituted or unsubstituted C₁-C₆ alkyl, or

R₃ and R₄ together with the carbon atoms to which they are attached form C₃-C₆ cycloalkyl.

In an embodiment, R₃ and R₄ are each independently selected from hydrogen, fluorine, and substituted or unsubstituted C₁-C₃ alkyl.

In an embodiment, R₃ is selected from hydrogen and substituted or unsubstituted C₁-C₆ alkyl.

In an embodiment, R₃ is selected from hydrogen, methyl, ethyl, propyl, isopropyl, butyl, isobutyl, sec-butyl, tert-butyl, n-pentyl, isopentyl, neopentyl, and hexyl, preferably hydrogen or methyl.

In an embodiment, R₄ is selected from hydrogen or fluorine, more preferably fluorine.

Preferably, y is 2.

The present disclosure provides the following compounds, and pharmaceutically acceptable salts and stereoisomers thereof:

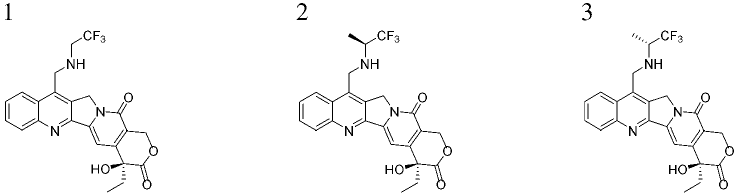

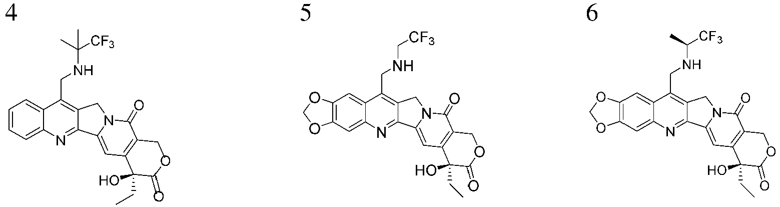

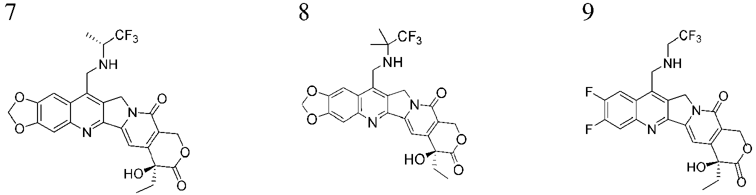

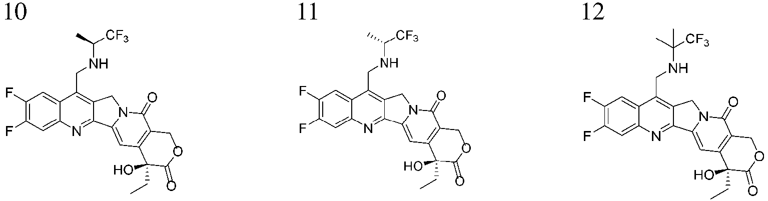

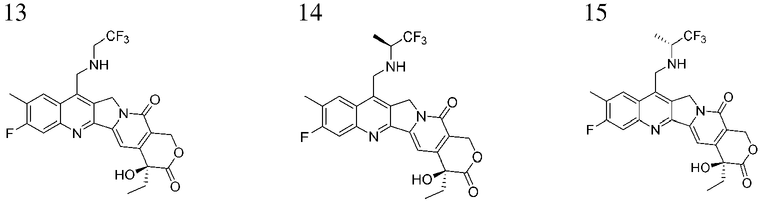

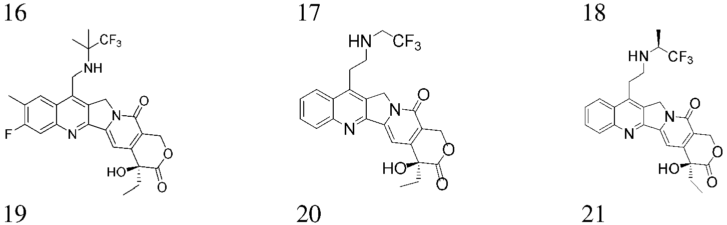

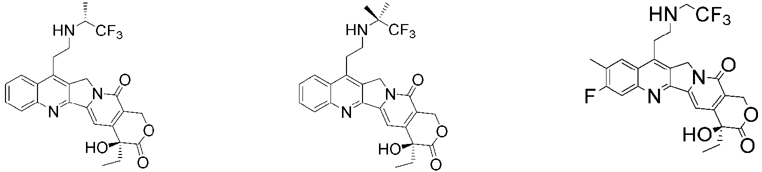

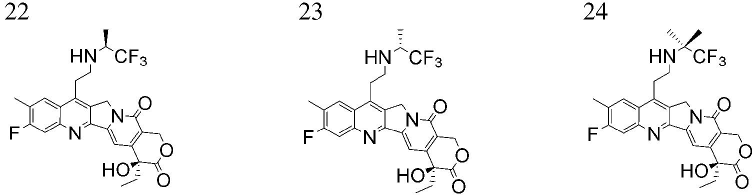

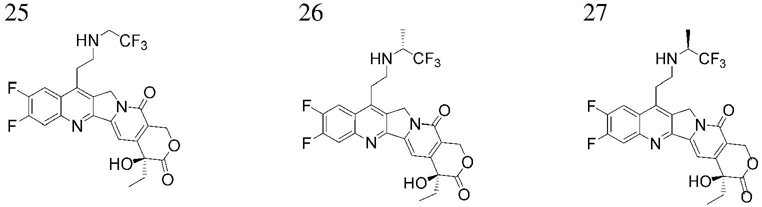

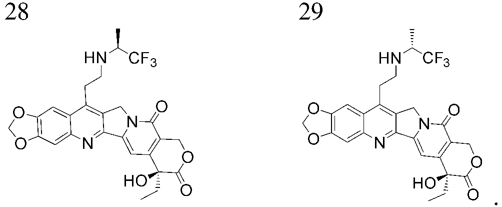

In another aspect, the present disclosure provides a method for preparing the compound described above, comprising the step of subjecting a 7-formylcamptothecin derivative and corresponding fluorine-substituted amine to reductive amination reaction.

In an embodiment, the 7-formylcamptothecin derivative is selected from any one of

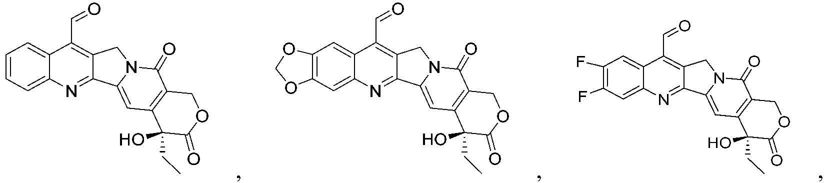

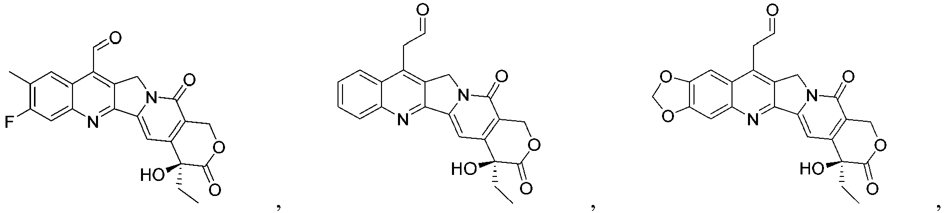

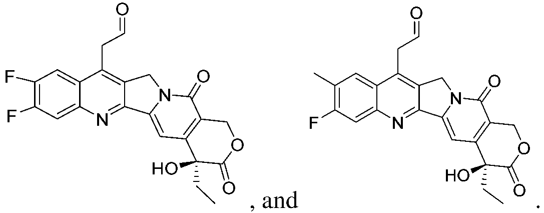

In an embodiment, the corresponding fluorine-substituted amine refers to at least one selected from compounds having the following structures, equivalents thereof, and pharmaceutically acceptable salts thereof: 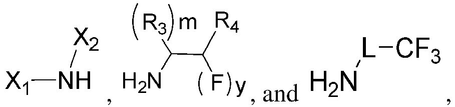 wherein, X₁, X₂, R₃, R₄, m, n, y, and L are each the same as defined above.

In an embodiment, the corresponding fluorine-substituted amine is selected from

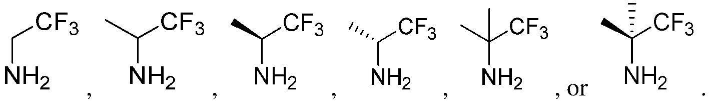

In another aspect, of the present disclosure provides a pharmaceutical composition, comprising: the compound or the pharmaceutically acceptable salt or stereoisomer thereof described above, and a pharmaceutically acceptable adjuvant.

In another aspect, of the present disclosure provides use of the compound or the pharmaceutically acceptable salt or stereoisomer thereof or the pharmaceutical composition described above in the manufacture of a medicament for treating a cancer.

In another aspect, of the present disclosure provides a method for treating a cancer, comprising the step of administering the compound or the pharmaceutically acceptable salt or stereoisomer thereof described above or the pharmaceutical composition described above to a subject.

In an embodiment, the administration amount of the compound or the pharmaceutically acceptable salt or stereoisomer thereof described above or the pharmaceutical composition described above is a therapeutically effective amount.

In an embodiment, the cancer is selected from solid tumor.

In an embodiment, the cancer is selected from one or more of esophageal cancer, gastric cancer, lung cancer, and breast cancer.

### DETAILED DESCRIPTION OF THE EMBODIMENTS

### I. Definitions

In the present disclosure, unless otherwise specified, the scientific and technical terms used herein have the meanings commonly understood by those skilled in the art. The relevant terms and laboratory procedures used herein are those commonly used and routinely practiced in the respective fields. Moreover, in order to well explain the present disclosure, definitions and explanations of related terms are provided below.

In the description herein, the term "some examples", "some implementations", or "some embodiments" describes a subset of all possible examples. However, it should be understood that "some examples" may refer to the same subset or different subsets of all possible examples and may be combined with one another in a non-conflicting situation.

As used herein and unless otherwise specified, the terms "comprising", "including", "having", and "containing", including grammatical equivalents thereof, should generally be understood to be open-ended and non-restrictive. For example, other unlisted elements or steps are not excluded.

The compound of the present disclosure may be asymmetric. For example, the compound of the present disclosure has one or more stereoisomers. Unless otherwise specified, all stereoisomers are included, such as enantiomers and diastereomers. The stereoisomers comprise geometric isomers (for example, cis/trans structures) and optical isomers (for example, enantiomers), which can be formulated into therapeutics in the forms of monomers, racemates, racemic mixtures and pharmaceutically acceptable salts thereof. In the present disclosure, the compound containing an asymmetric carbon atom can be isolated in an optically active pure form or in a racemic form. The optically active pure form can be resolved from a racemic mixture, or synthesized by using a chiral raw material or a chiral reagent. Racemates, diastereomers, and enantiomers are all included within the scope of the present disclosure.

A numerical range herein refers to each integer falling within the given range. For example, "C₁-C₈" means that the group may have 1, 2, 3, 4, 5, 6, 7, or 8 carbon atoms, and "C₃-C₆" means that the group may have 3, 4, 5, or 6 carbon atoms.

The term "substituted" means that any one or more hydrogen atoms on a specified atom or group are replaced by a substituent, provided that the valence of the specified atom or group remains normal and the substituted compound is stable. Unless otherwise specified, the type and number of substituents can be arbitrary on the premise of chemical feasibility.

When any variable (such as Rₙ) occurs more than once in a composition or structure of a compound, such variable shall be defined independently at each occurrence. Thus, for example, if a group is substituted with one to four R groups, the group may optionally be substituted with up to four R groups, and R is independently selected at each occurrence. Further, combinations of substituents and/or variants thereof are permissible only if such combinations result in stable compounds.

The term "alkyl" refers to saturated aliphatic hydrocarbyl, which is a linear or branched group containing 1 to 20 carbon atoms, preferably alkyl containing 1 to 8 carbon atoms, more preferably alkyl containing 1 to 6 carbon atoms, most preferably alkyl containing 1 to 3 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, and various branched isomers thereof. More preferably, the alkyl is lower alkyl containing 1 to 6 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, and 2,3-dimethylbutyl. The alkyl may be substituted or unsubstituted. When the alkyl is substituted, a substituent may be attached at any available attachment site. 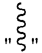 indicates an attachment site for a chemical bond.

The term "pharmaceutically acceptable salt" refers to a salt formed by a corresponding amine compound with an inorganic acid or an organic acid, a salt formed by a corresponding carboxylic acid compound with an alkali metal or alkaline earth metal, or a salt formed by a corresponding carboxylic acid compound with an organic amine. The inorganic acid includes, but is not limited to, hydrochloric acid, hydrobromic acid, hydroiodic acid, sulfuric acid, and phosphoric acid. The organic acid includes, but is not limited to, acetic acid, propionic acid, butyric acid, benzoic acid, methanesulfonic acid, benzenesulfonic acid, p-toluenesulfonic acid, oxalic acid, succinic acid, lactic acid, citric acid, succinic acid, gluconic acid, maleic acid, fumaric acid, and tartaric acid. The alkali metal or alkaline earth metal salt includes, but is not limited to, sodium, potassium, calcium, and magnesium salts. The organic amine salt includes, but is not limited to, salts of ammonia, methylamine, ethylamine, propylamine, isopropylamine, dimethylamine, diethylamine, trimethylamine, triethylamine, tert-butylamine, ethylenediamine, ethanolamine, diethanolamine, triethanolamine, morpholine, piperidine, piperazine, and amino acids.

The drug or pharmaceutical composition of the present disclosure may be administered orally, topically, parenterally, or mucosally (for example, buccally, by inhalation, or rectally) in a dosage unit formulation comprising a conventional non-toxic pharmaceutically acceptable carrier. The oral administration is generally preferred. The active agent may be administered orally in a form of a capsule, a tablet, etc.

The term "treating" includes inhibiting, alleviating, preventing, or eliminating one or more symptoms or side effects associated with a disease, a disorder, or a condition to be treated.

The term "inhibiting" is used relative to a control. Those skilled in the art will readily determine an appropriate control for each experiment. For example, a reduced response in a subject or cell treated with the compound is compared with a response in a subject or cell not treated with the compound.

The term "pharmaceutical composition" refers to a composition comprising a compound disclosed herein or a pharmaceutically acceptable salt thereof, together with at least one pharmaceutically acceptable ingredient selected according to the administration route and dosage form characteristics, including but not limited to a carrier, a diluent, an adjuvant, an excipient, a preservative, a filler, a disintegrant, a wetting agent, an emulsifier, a suspending agent, a sweetener, a flavoring agent, a fragrance, an antibacterial agent, an antifungal agent, a lubricant, a dispersant, a thermosensitive material, a temperature regulator, an adhesive, a stabilizer, and a suspending aid.

The term "effective amount" or "therapeutically effective amount" refers to a sufficient amount of a drug or an agent that is non-toxic but can allow a desired effect. In an embodiment of the present disclosure, when a patient is treated according to the present disclosure, a dosage of a given drug depends on many factors, such as a specific dosing regimen, a type and severity of a disease or condition, and uniqueness of a subject or host in need of the treatment (such as a body weight). However, depending on the particular surrounding circumstances, including, for example, a specific drug employed, a route of administration, a condition to be treated, and a subject or host to be treated, an administered dosage can be routinely determined by a method known in the art. The administered dosage for adult therapeutic use is typically 0.02 mg/day to 5,000 mg/day, for example, about 1 mg/day to 1,500 mg/day. A required dose may conveniently be presented as a single dose, or as divided doses administered simultaneously (or within a short period of time) or at an appropriate interval, for example, two, three, four, or more divided doses daily. Those skilled in the art will appreciate that, although the above dosage ranges are provided, the specific effective dosage may be appropriately adjusted according to conditions of a patient and diagnosis of a physician.

The term "subject" refers to a mammal that is assessed for treatment and/or is treated. In an embodiment, the mammal is a human being. The term "subject" includes, but is not limited to, an individual suffering from a cancer, an individual suffering from an autoimmune disease, and an individual suffering from a pathogenic infection. The subject may be a human being, but also includes other mammals, especially those serving as laboratory models for human diseases, such as mice and rats.

### II. Examples

To make the objectives, technical solutions, and advantages of the present disclosure clear, the present disclosure will be described in further detail below. The described examples should not be construed as limiting the present disclosure. All other examples obtained by those of ordinary skill in the art without creative effort shall fall within the scope of protection of the present disclosure.

Before the examples of the present disclosure are further described in detail, the terms and expressions involved in the examples of the present disclosure have been explained. The terms and expressions involved in the examples of the present disclosure are subject to such explanations.

The raw materials and devices used in the specific examples of the present disclosure are all known products, which can be commercially available products.

### Example 1: 7-(N-(2',2',2'-trifluoroethyl)amino)methylcamptothecin (1)

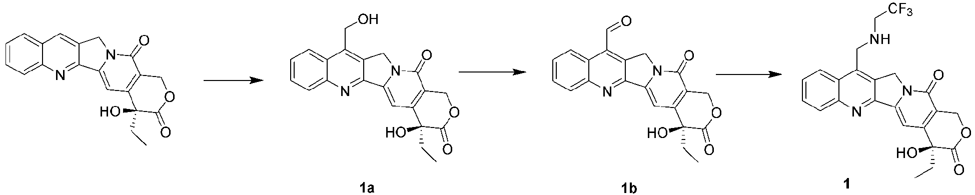

Camptothecin (10 g, 28.62 mmol), methanol (330 mL), and water (275 mL) were added to a reaction flask. 75% sulfuric acid (275 mL) was added dropwise under stirring. FeSO₄·7H₂O (7.96 g, 28.62 mmol) was then added. Then, a 30% H₂O₂ aqueous solution (440.00 mL) was added dropwise under stirring. The reaction solution was further stirred at room temperature for 18 h, and then poured into ice water to precipitate a solid. The solid was collected by filtration and then dried to afford 7-hydroxymethylcamptothecin **1a** (7 g, yield: 51.57%). LCMS: [M+1]⁺ 379.12(theoretical value: 378.12), which was used directly in the next step without further purification.

**1a** (9.5 g, 25.04 mmol), sodium bicarbonate (8.41 g, 100.16 mmol), and dichloromethane (DCM) (190 mL) were added to a reaction flask. Under ice-bath cooling and stirring, Dess-Martin periodinane (15.93 g, 37.56 mmol) was added. The reaction solution was stirred at room temperature overnight, and then poured into water to precipitate a solid. The solid was collected by filtration and dried to afford camptothecin-7-carbaldehyde **1b** (9 g, yield: 76%). [M+1]⁺ 377.21 (theoretical value: 376.11), which was used directly in the next step without further purification.

**1b** (1.5 g, 4.0 mmol), 2,2,2-trifluoroethylamine (1.18 g, 11.9 mmol), DCM (60 mL), and NaBH₃CN (0.75 g, 11.9 mmol) were added to a reaction flask. The reaction solution was stirred at room temperature for 3 h, concentrated, and then purified by silica gel column chromatography to afford 7-(N-(2',2',2'-trifluoroethyl)amino)methylcamptothecin **1** (376 mg, yield: 20%, HPLC: 98.5%). LCMS: [M+1]⁺ 460.39 (theoretical value: 459.43); ¹H NMR (500 MHz, DMSO-d₆) δ 8.44 (d, *J*=8.5 Hz, 1H), 8.27-8.18 (m, 1H), 7.91 (ddd, *J*=8.3, 6.8, 1.3 Hz, 1H), 7.79 (ddd, *J*=8.3, 6.8, 1.3 Hz, 1H), 7.37 (s, 1H), 5.59 (s, 2H), 5.50-5.36 (m, 3H), 4.76 (s, 3H), 4.07 (q, *J*=9.6 Hz, 2H), 1.96-1.85 (m, 2H), 0.90 (t, *J*=7.3 Hz, 3H).

### Example 2: 7-(N-(S)-(1',1',1'-trifluoropropan-2'-yl)amino)methylcamptothecin (2)

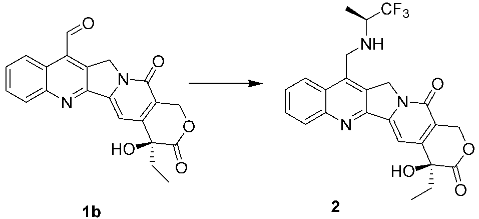

**1b** (2 g, 5.3 mmol), (S)-1,1,1-trifluoropropan-2-amine (2.38 g, 15.9 mmol, 2.07 mL), DCM/dimethyl sulfoxide (DMSO) (4:1, 20 mL), and NaBH₃CN (1 g, 15.9 mmol) were added to a reaction flask. The reaction solution was stirred at room temperature for 2 h, concentrated, and then purified by silica gel column chromatography to afford 7-(N-(S)-(1',1',1'-trifluoropropan-2'-yl)amino)methylcamptothecin **2** (900 mg, yield: 31.5%, HPLC: 95%). LCMS: [M+H]⁺ 474.60 (theoretical value: 473.45); ¹H NMR (500 MHz, DMSO-d₆) δ 8.33 (dd, *J*=8.6, 1.4 Hz, 1H), 8.15 (dd, *J*=8.5, 1.3 Hz, 1H), 7.83 (ddd, *J*=8.3, 6.8, 1.3 Hz, 1H), 7.70 (ddd, *J*=8.3, 6.8, 1.3 Hz, 1H), 7.33 (s, 1H), 6.53 (s, 1H), 5.43 (s, 2H), 5.36 (d, *J*=1.7 Hz, 2H), 4.47 (d, *J*=13.7 Hz, 1H), 4.38 (d, *J*=13.7 Hz, 1H), 3.53 (p, *J*=7.3 Hz, 1H), 1.88 (dh, *J*=21.5, 7.2 Hz, 2H), 1.27 (d, *J*=6.7 Hz, 3H), 0.90 (t, *J*=7.3 Hz, 3H); ¹³C NMR (126 MHz, DMSO-d₆) δ 172.96, 157.26, 152.61, 150.48, 148.89, 146.04, 141.67, 130.41, 130.07, 129.33, 128.93, 127.89, 127.24, 126.68, 125.11, 119.51, 97.07, 72.86, 65.74, 54.83, 50.49, 45.81, 30.78, 14.52, 14.50, 14.48, 8.24.

### Example 3: 7-(N-(R)-(1',1',1'-trifluoropropan-2'-yl)amino)methylcamptothecin (3)

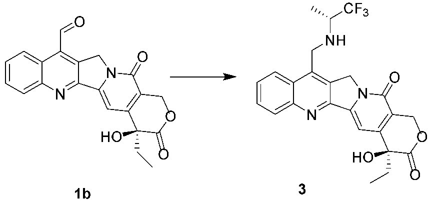

**1b** (200 mg, 530 µmol), (R)-1,1,1-trifluoropropan-2-amine (181.40 mg, 1.21 mmol), DCM/DMSO (4:1, 10 mL), and NaBH₃CN (100 mg, 15.9 mmol) were added to a reaction flask. The reaction solution was stirred at room temperature for 2 h, concentrated, and then purified by silica gel column chromatography to afford 7-(N-(R)-(1',1',1'-trifluoropropan-2'-yl)amino)methylcamptothecin **3** (40 mg, yield: 15%, HPLC: 97%). LCMS: [M+H]⁺ 474.60 (theoretical value: 473.45); ¹H NMR (500 MHz, DMSO-d₆) δ 8.34 (d, *J*=8.5 Hz, 1H), 8.17 (d, *J*=8.5 Hz, 1H), 7.85 (t, *J*=8.5 Hz, 1H), 7.72 (t, *J*=8.5, 1H), 7.35 (s, 3H), 5.46 (s, 2H), 5.38 (s, 2H), 4.50 (d, *J*=13.6 Hz, 1H), 4.40 (d, *J*=13.7 Hz, 1H), 3.55 (p, *J*=7.2 Hz, 2H), 1.99-1.83 (m, 2H), 1.30 (d, *J*=6.7 Hz, 3H), 0.92 (t, *J*=7.3 Hz, 3H); ¹³C NMR (126 MHz, DMSO-d₆) δ 172.94, 157.25, 152.60, 150.49, 148.88, 146.03, 141.47, 130.41, 130.06, 129.38, 128.84, 127.89, 127.24, 126.59, 125.12, 119.51, 97.09, 72.87, 65.74, 55.29, 55.07, 54.85, 54.63, 50.50, 45.75, 30.80, 14.41, 12.46, 8.24.

### Example 4: 7-(N-(2'-trifluoromethylpropan-2'-yl)amino)methylcamptothecin (4)

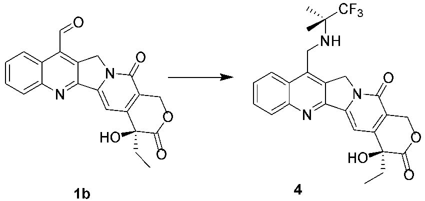

**1b** (400 mg, 1.06 mmol), 2-trifluoromethylpropan-2-amine hydrochloride (520.13 mg, 3.18 mmol), DCM (24 mL), DMSO (4 mL), and NaBH₃CN (200 mg, 3.18 mmol) were added to a reaction flask. The reaction solution was stirred at room temperature for 3 h, concentrated, and then purified by silica gel column chromatography to afford 7-(N-(2'-trifluoromethylpropan-2'-yl)amino)methylcamptothecin **4** (42 mg, yield: 7.5%, HPLC: 94%). LCMS: [M+H]⁺ 488.28 (theoretical value: 487.48); ¹H NMR (500 MHz, DMSO-*d*₆) δ 8.30 (d, *J*=8.4 Hz, 1H), 8.17 (d, *J*=8.3 Hz, 1H), 7.86 (ddd, *J*=8.4, 6.6, 1.3 Hz, 1H), 7.74 (ddd, *J*=8.4, 6.7, 1.3 Hz, 1H), 7.34 (s, 1H), 5.43 (d, *J*=14.0 Hz, 4H), 4.42 (s, 2H), 1.89 (ddt, *J*=17.9, 14.1, 7.1 Hz, 2H), 1.41 (s, 6H), 0.89 (t, *J*=7.3 Hz, 3H).

### Example 5: 7-(N-(2',2',2'-trifluoroethyl)amino)methyl-10,11-methylenedioxycamptothecin (5)

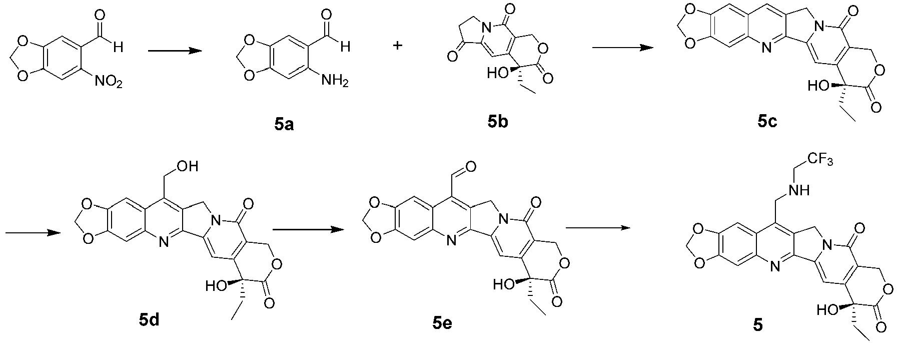

6-nitropiperonal (25 g, 127.46 mmol) was dissolved in methanol (250 mL) in a reaction flask, and 10% Pd/C (2.49 g, 23.37 mmol) was added. The reaction solution was stirred under a hydrogen balloon for 16 h, filtered, and concentrated to afford a product **5a** (20 g, 116.76 mmol, yield: 91.60%, HPLC: 97%). LCMS: [M+1]⁺ 166.91 (theoretical value: 165.04).

A tricyclic intermediate **5b** (26.51 g, 100.31 mmol), toluene (200 mL), **5a** (20 g, 120.37 mmol), and pyridinium p-toluenesulfonate (PPTS) (10.08 g, 40.12 mmol) were added to a reaction flask. The reaction solution was heated to 110°C under stirring to react for 16 h, and concentrated under reduced pressure. Acetone (50 mL) was added to precipitate a solid. The solid was collected by filtration, washed with methanol, and dried to afford **5c** (33 g, yield: 80.29%, HPLC: 96%). LCMS: [M+1]⁺ 393.39 (theoretical value: 392.37); ¹H NMR (500 MHz, DMSO-d6) δ 8.46 (s, 1H), 7.51 (s, 2H), 7.26 (s, 1H), 6.28 (s, 2H), 5.42 (s, 2H), 5.22 (s, 2H), 1.87 (qd, *J*=14.7, 7.3 Hz, 2H), 0.89 (t, *J*=7.4 Hz, 3H).

**5c** (11 g, 27.96 mmol), methanol (330 mL), H₂O (275 mL), H₂SO₄ (75%) (2.74 g, 27.96 mmol, 275 mL), and FeSO₄·7H₂O (8.8 g, 31.65 mmol) were added to a reaction flask. At 0°C, a 30% H₂O₂ aqueous solution (55.50 g, 1.63 mol, 50 mL) was slowly added dropwise. The reaction solution was stirred at room temperature for 16 h, and then H₂O (500 mL) was added to precipitate a solid. The solid was collected by filtration, washed with acetonitrile (ACN) and methyl tert-butyl ether, and dried to afford a brown solid product **5d** (8.3 g, yield: 63.10%, HPLC: 94%). LCMS: [M+1]⁺ 423.39 (theoretical value: 422.39); ¹H NMR (500 MHz, DMSO-d₆) δ 7.39 (s, 1H), 7.34 (s, 1H), 7.22 (s, 1H), 6.25 (d, *J*=5.2 Hz, 2H), 5.45-5.35 (m, 2H), 5.12 (s, 2H), 5.04 (s, 2H), 1.94-1.80 (m, *J*=7.1 Hz, 2H), 0.90 (t, *J*=7.2 Hz, 3H).

**5d** (5 g, 11.81 mmol), DCM (100 mL), and DMSO (20 mL) were added to a reaction flask. At 0°C, Dess-Martin periodinane (10.02 g, 23.62 mmol) was added dropwise under stirring. The reaction solution was stirred at room temperature for 16 h, then concentrated under reduced pressure, and purified by silica gel column chromatography to afford a yellow solid product **5e** (2.7 g, yield: 49.92%, HPLC: 92%). LCMS: [M+1]⁺ 421.36 (theoretical value: 420.38), which was used directly in the next step.

**5e** (2.7 g, 6.41 mmol), 2,2,2-trifluoroethylamine (2.60 g, 19.22 mmol, 2.09 mL), DCM (60 mL), DMSO (10 mL), and NaBH₃CN (1.21 g, 19.22 mmol) were added to a reaction flask. The reaction solution was stirred at room temperature for 3 h, then concentrated under reduced pressure, and purified by silica gel column chromatography to afford a yellow solid product **5** (1.5 g, yield: 42.70%, HPLC: 95%). LCMS: [M+1]⁺ 504.28 (theoretical value: 503.43); ¹H NMR (500 MHz, DMSO-d₆) δ 7.66 (s, 1H), 7.50 (s, 1H), 7.24 (s, 1H), 6.48 (s, 1H), 6.29 (d, *J*=2.3 Hz, 2H), 5.42 (s, 2H), 5.29 (s, 2H), 4.27 (d, *J*=6.6 Hz, 2H), 3.40 (dd, *J*=10.2, 7.2 Hz, 2H), 3.09 (p, *J*=7.0 Hz, 1H), 1.86 (dh, *J*=21.5, 7.3 Hz, 2H), 0.88 (t, *J*=7.3 Hz, 3H).

### Example 6: 7-(N-(S)-(1',1',1'-trifluoropropan-2'-yl)amino)methyl-10,11-methylenedioxycamptothecin (6)

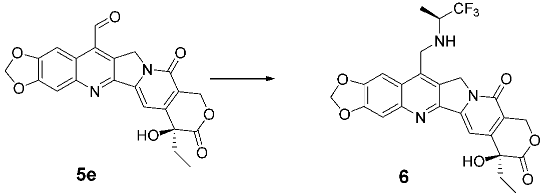

**5e** (0.85 g, 1.21 mmol), (S)-1,1,1-trifluoroisopropylamine (352.27 mg, 2.42 mmol), DCM (40 mL), and DMSO (10 mL) were added to a reaction flask, and stirred for 30 min. NaBH₃CN (114.09 mg, 1.82 mmol) was added. The reaction solution was further stirred at room temperature for 2 h, then concentrated under reduced pressure, and purified by silica gel column chromatography to afford a solid product **6** (0.54 g, yield: 77%, HPLC: 95%). LCMS [M+H]⁺ 518.2 (theoretical value: 517.15); ¹H NMR (600 MHz, DMSO-*d*₆) δ 7.67 (s, 1H), 7.50 (s, 1H), 7.24 (s, 1H), 6.49 (s, 1H), 6.29 (d, *J*=2.1 Hz, 2H), 5.42 (s, 2H), 5.31 (d, *J*=3.7 Hz, 2H), 4.35 (dd, *J*=13.6, 5.9 Hz, 1H), 4.24 (dd, *J*=13.4, 7.8 Hz, 1H), 3.49 (h, *J*=7.3 Hz, 1H), 2.80 (q, *J*=7.1 Hz, 1H), 1.87 (m, *J*=21.6, 14.0, 7.2 Hz, 2H), 1.25 (d, *J*=6.7 Hz, 3H), 0.88 (t, *J*=7.3 Hz, 3H).

### Example 7: 7-(N-(R)-(1',1',1'-trifluoropropan-2'-yl)amino)methyl-10,11-methylenedioxycamptothecin (7)

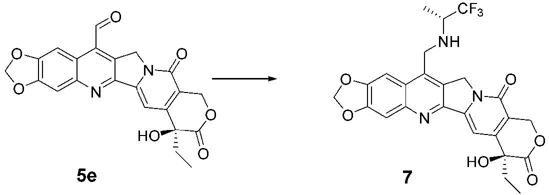

**5e** (500 mg, 1.19 mmol), (R)-1,1,1-trifluoropropan-2-amine (201.27 mg, 1.35 mmol), DCM (8 mL), and DMSO (2 mL) were added to a reaction flask and stirred for 30 min. NaBH3CN (111.85 mg, 1.78 mmol) was added. The reaction solution was further stirred at room temperature for 2 h, then concentrated under reduced pressure, and purified by silica gel column chromatography to afford a solid product **7** (200 mg, yield: 30%, HPLC: 94%). LCMS: [M+H]⁺ 518.26 (theoretical value: 517.46); ¹H NMR (500 MHz, DMSO-d₆) δ 7.68 (s, 1H), 7.50 (s, 1H), 7.24 (s, 1H), 6.48 (s, 1H), 6.29 (d, *J*=2.6 Hz, 2H), 5.42 (s, 2H), 5.32 (d, *J*=4.1 Hz, 2H), 4.35 (dd, *J*=13.5, 5.8 Hz, 1H), 4.24 (dd, *J*=13.5, 7.8 Hz, 1H), 3.49 (h, *J*=7.5 Hz, 1H), 2.80 (q, *J*=7.1 Hz, 1H), 1.86 (ddq, *J*=21.3, 14.3, 7.2 Hz, 2H), 1.25 (d, *J*=6.8 Hz, 3H), 0.88 (t, *J*=7.3 Hz, 3H).

### Example 8: 7-(N-(2'-trifluoromethylpropan-2'-yl)amino)methyl-10,11-methylenedioxycamptothecin (8)

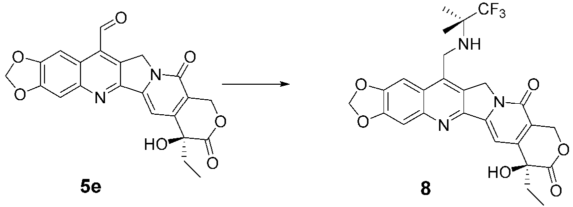

**5e** (200 mg, 474.63 µmol), 2-trifluoromethylpropan-2-amine (116.45 mg, 711.95 µmol), DCM (8 mL), and DMSO (2 mL) were added to a reaction flask and stirred for 30 min. NaBH₃CN (44.74 mg, 711.95 µmol) was added. The reaction solution was further stirred at room temperature for 2 h, then concentrated under reduced pressure, and purified by silica gel column chromatography to afford a solid product 7 (200 mg, yield: 7.5%, HPLC: 95%). LCMS: [M+H]⁺ 532.28 (theoretical value: 531.49); ¹H NMR (600 MHz, DMSO-d₆) δ 7.62 (s, 1H), 7.52 (s, 1H), 7.25 (s, 1H), 6.49 (s, 1H), 6.30 (d, *J*=1.9 Hz, 2H), 5.42 (s, 2H), 5.35 (s, 2H), 4.26 (s, 2H), 2.84 (s, 1H), 1.93-1.81 (m, 3H), 1.36 (s, 6H), 1.24 (s, 1H), 0.88 (t, *J*=7.3 Hz, 3H).

### Example 9: 7-(N-(2',2',2'-trifluoroethyl)amino)methyl-10,11-difluorocamptothecin (9)

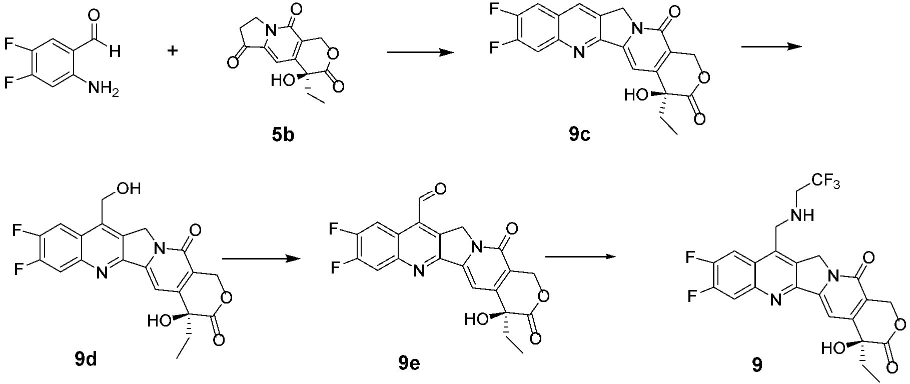

2-nitro-4,5-difluorobenzaldehyde (13.6 g, 86.01 mmol), pyridinium p-toluenesulfonate (PPTS) (196.49 g, 781.89 mmol), and the tricyclic intermediate **5b** (20.58 g, 77.89 mmol) were added to a reaction flask. The reaction solution was heated to 130°C under stirring to react for 2 hours, and cooled to room temperature. Methanol (100 mL) was added. The resulting mixture was stirred at room temperature overnight to precipitate a solid. The solid was collected by filtration and dried to afford a brown solid **9c** (23 g, yield: 71.76%, HPLC: 94%). LCMS: [M+H]⁺ 385.20 (theoretical value: 384.09), which was used directly in the next step.

**9c** (15 g, 38.93 mmol), methanol (450 mL), and H₂O (325 mL) were added to a reaction flask. At 0°C, 75% H₂SO₄ (3.82 g, 38.93 mmol, 325 mL) and FeSO₄·7H₂O (10.82 g, 38.93 mmol) were added dropwise under stirring. At 0°C, a 30% H₂O₂ aqueous solution (83.25 g, 2.45 mol, 75 mL) was slowly added dropwise. The reaction solution was stirred at room temperature for 16 h, and then H₂O (650 mL) was added to precipitate a solid. The solid was collected by filtration, washed with ACN and methyl tert-butyl ether, and dried to afford a brown solid product **9d** (10 g, yield: 40.20%). LCMS: [M+1]⁺ 415.24 (theoretical value: 414.36).

**9d** (6 g, 14.45 mmol), DCM (120 mL), and DMSO (20 mL) were added to a reaction flask. At 0°C, Dess-Martin periodinane (12.25 g, 28.89 mmol) was added dropwise under stirring. The reaction solution was stirred at room temperature for 16 h, and then poured into water to precipitate a solid. The solid was collected by filtration and dried to afford **9e** (9 g, yield: 52.76%). LCMS: [M+1]⁺ 413.35 (theoretical value: 412.35).

**9e** (1 g, 2.42 mmol), 2,2,2-trifluoroethylamine hydrochloride (983.54 mg, 7.26 mmol), DCM (30 mL), DMSO (5 mL), and NaBH₃CN (0.6 g, 9.62 mmol) were added to a reaction flask. The reaction solution was stirred at room temperature for 3 h, then concentrated under reduced pressure, and purified by silica gel column chromatography to afford a yellow solid product **9** (440 mg, yield: 35.17%, HPLC: 96%). LCMS: [M+1]⁺496.26 (theoretical value: 495.41); ¹H NMR (500 MHz, DMSO-d₆) δ 8.30 (dd, *J*=12.3, 8.7 Hz, 1H), 8.10 (dd, *J*=11.4, 8.0 Hz, 1H), 7.28 (s, 1H), 6.52 (s, 1H), 5.41 (s, 2H), 5.29 (s, 2H), 4.33 (d, *J*=6.5 Hz, 2H), 3.42 (dd, *J*=10.5, 7.5 Hz, 2H), 3.17 (p, *J*=7.0 Hz, 1H), 1.87 (dh, *J*=14.3, 7.2 Hz, 2H), 0.89 (t, *J*=7.3 Hz, 3H).

### Example 10: 7-(N-(S)-(1',1',1'-trifluoropropan-2'-yl)amino)methyl-10,11-difluorocamptothecin (10)

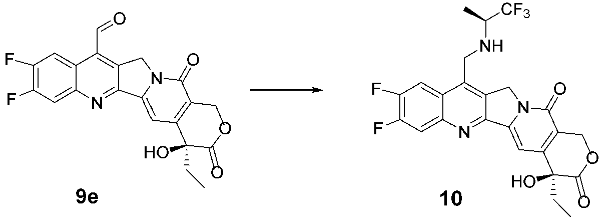

**9e** (1.5 g, 3.64 mmol), (S)-1,1,1-trifluoropropan-2-amine hydrochloride (1.63 g, 10.92 mmol), DCM (50 mL), DMSO (8 mL), and NaBH₃CN (686 mg, 10.92 mmol) were added to a reaction flask. The reaction solution was stirred at room temperature for 2 h. Then, a saturated NaHCO₃ aqueous solution (50 mL) was added to precipitate a solid. The solid was collected by filtration and purified by silica gel column chromatography to afford a solid product **10** (500 mg, yield: 27%, HPLC: 97%). LCMS: [M+H]⁺ 510.26 (theoretical value: 509.14); ¹H NMR (500 MHz, DMSO-d₆) δ 8.28 (dd, *J*=12.3, 8.7 Hz, 1H), 8.10 (dd, *J*=11.4, 8.0 Hz, 1H), 7.28 (s, 1H), 6.51 (s, 1H), 5.41 (s, 2H), 5.28 (s, 2H), 4.40 (dd, *J*=14.0, 5.9 Hz, 1H), 4.28 (dd, *J*=14.0, 7.5 Hz, 1H), 3.51 (q, *J*=7.3 Hz, 1H), 2.87 (q, *J*=7.1 Hz, 1H), 1.87 (p, *J*=7.2 Hz, 2H), 1.26 (d, *J*=6.8 Hz, 3H), 0.88 (t, *J*=7.3 Hz, 3H); ¹³C NMR (126 MHz, DMSO-*d*₆) δ 172.87, 157.11, 153.27, 150.42, 146.43, 146.34, 145.47, 141.83, 129.68, 124.77, 124.70, 119.88, 116.17, 116.04, 111.88, 111.73, 97.29, 72.80, 65.71, 54.94, 54.72, 50.38, 46.03, 30.81.

### Example 11: 7-(N-(R)-(1',1',1'-trifluoropropan-2'-yl)amino)methyl-10,11-difluorocamptothecin (11)

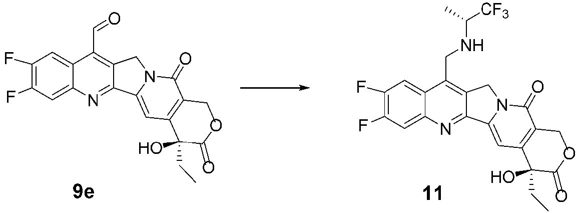

**9e** (500 mg, 1.21 mmol), (R)-1,1,1-trifluoropropan-2-amine hydrochloride (410.36 mg, 2.74 mmol), DCM (8 mL), DMSO (2 mL), and NaBH₃CN (228.05 mg, 3.63 mmol) were added to a reaction flask. The reaction solution was stirred at room temperature for 2 h. Then, a saturated NaHCO₃ aqueous solution (50 mL) was added to precipitate a solid. The solid was collected by filtration and purified by silica gel column chromatography to afford a yellow solid product **11** (150 mg, yield: 23.08%, HPLC: 95%). LCMS: [M+H]⁺ 510.43 (theoretical value: 509.43); ¹H NMR (600 MHz, DMSO-d₆) δ 8.30 (dd, *J*=12.3, 8.7 Hz, 1H), 8.12 (dd, *J*=11.4, 8.0 Hz, 1H), 7.29 (s, 1H), 6.53 (s, 1H), 5.42 (s, 2H), 5.30 (d, *J*=6.2 Hz, 2H), 4.41 (dd, *J*=13.9, 5.9 Hz, 1H), 4.28 (dd, *J*=13.9, 7.6 Hz, 1H), 3.50 (h, *J*=7.3 Hz, 1H), 2.88 (td, *J*=7.5, 5.9 Hz, 1H), 1.87 (dp, *J*=21.4, 7.1 Hz, 2H), 1.27 (d, *J*=6.8 Hz, 3H), 1.21 (q, *J*=5.2, 4.4 Hz, 1H), 0.89 (t, *J*=7.3 Hz, 3H); ¹³C NMR (151 MHz, DMSO-d₆) δ 172.86, 157.12, 153.28, 153.27, 152.65, 152.54, 150.97, 150.86, 150.63, 150.53, 150.44, 148.97, 148.87, 146.42, 146.34, 145.50, 141.83, 141.79, 130.50, 130.08, 129.71, 128.63, 126.76, 124.88, 124.80, 124.74, 119.86, 116.15, 116.04, 111.91, 111.78, 97.30, 72.80, 65.70, 55.10, 54.91, 54.73, 54.54, 50.40, 46.03, 31.73, 30.80, 29.48, 29.28, 29.19, 29.02, 26.99, 25.57, 22.54, 14.42, 14.41, 8.19.

### Example 12: 7-(N-(2'-trifluoromethylpropan-2'-yl)amino)methyl-10,11-difluorocamptothecin (12)

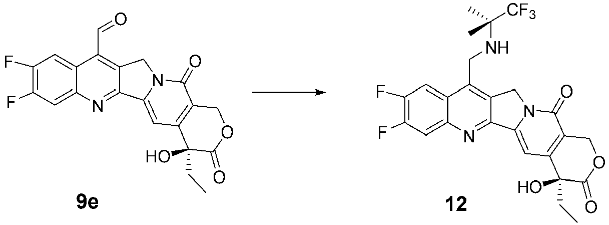

**9e** (350 mg, 846.74 µmol), 2-trifluoromethylisopropylamine hydrochloride (15.50 mg, 2.54 mmol), DCM (18 mL), DMSO (3 mL), and NaBH₃CN (159.63 mg, 2.54 mmol) were added to a reaction flask. The reaction solution was stirred at room temperature for 3 h. Then, a saturated aqueous NaHCO₃ solution (50 mL) was added to precipitate a solid. The solid was collected by filtration and purified by silica gel column chromatography to afford a yellow solid product **12** (140 mg, yield: 29.00%). LCMS: [M+H]⁺ 524.30 (theoretical value: 523.46); ¹H NMR (600 MHz, DMSO-d₆) δ 8.23 (dddt, *J*=56.2, 11.0, 8.1, 2.5 Hz, 2H), 7.32 (t, *J*=1.2 Hz, 1H), 6.54 (s, 1H), 5.50-5.28 (m, 4H), 4.33 (d, *J*=7.3 Hz, 2H), 2.94 (t, *J*=7.4 Hz, 1H), 1.87 (ddp, *J*=21.4, 14.5, 7.3 Hz, 2H), 1.37 (s, 6H), 0.88 (t, *J*=7.3 Hz, 3H).

### Example 13: 7-(N-(2',2',2-trifluoroethyl)amino)methyl-10-methyl-11-fluorocamptothecin (13)

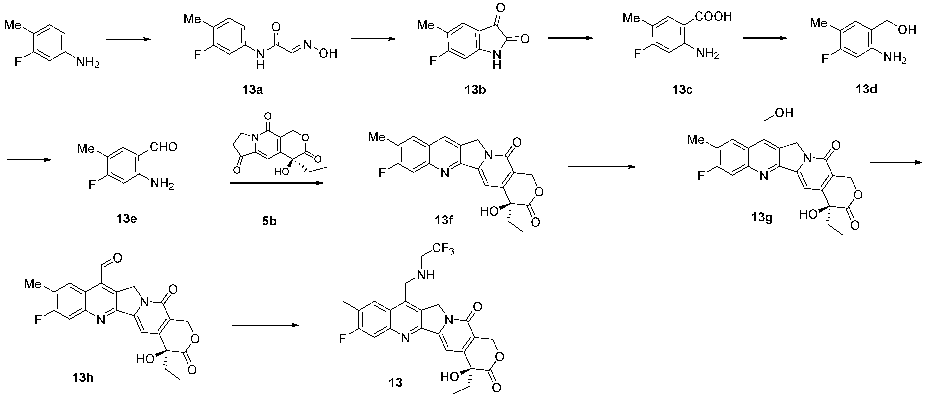

Hydroxylamine sulfate (444.0 g, 2.4 mol), anhydrous sodium sulfate (494.0 g, 3.5 mol), and a solution of chloral hydrate (65.0 g, 519.4 mmol) in water (1,600 mL) were added to a reaction flask and stirred for dissolution. 3-fluoro-4-methylaniline (65.0 g, 438.8 mmol) and 1 N HCl (550.0 mL) were added. The reaction solution was heated to 45°C under stirring to react for 2 h, further heated to 75°C to react for 1 h, and cooled to precipitate a solid. The solid was collected by filtration and dried to afford **13a** (96 g, yield: 94%, HPLC: 95%). LCMS: [M+H]⁺ 197.20 (theoretical value: 196.06); ¹H NMR (300 MHz, DMSO-d6) δ 2.08 (d, *J*=1.0 Hz, 3H), 7.12 (t, *J*=8.8 Hz, 1H), 7.25 (dd, *J*=2. 1,8. 2 Hz, 1H), 7.36 (dd, *J*=1.6, 12.4 Hz, 1H), 10.18 (s, 1H), 12.11 (s, 1H).

98% concentrated sulfuric acid (1,200 mL) and **13a** (96 g, 438.0 mmol) were added to a reaction flask. The reaction solution was heated to 80°C under stirring to react for 4 h, then cooled to room temperature, and poured into ice water (2,400 mL) to precipitate a solid. The solid was collected by filtration, washed multiple times with water, and dried (60°C) to afford an intermediate **13b** (85 g, yield: 94%, HPLC: 98%). LCMS: [M+H]⁺ 180.01 (theoretical value: 179.04); ¹H NMR (500 MHz, CDCl₃) δ 7.70-7.65 (m, 2H), 7.48 (d, *J*=7.9 Hz, 2H), 2.28 (s, 5H).

**13b** (85.0 g, 420.5 mmol), KOH (29.0 g, 685.3 mmol), KCl (97.8 g, 1,300 mmol), and water (1,700.0 mL) were added to a reaction flask. At 0°C, a 30% H₂O₂ aqueous solution (85.0 g) was added dropwise under stirring. The reaction solution was stirred at room temperature overnight, then cooled in an ice bath, and adjusted with 4 N HCl to a pH of 4 to 6, such that a solid was precipitated. The solid was collected by filtration and dried 48°C for 16 h to afford **13c** (48.5 g, yield: 60.4%, HPLC: 95%). LCMS: [M+H]⁺ 170.10 (theoretical value: 169.05); ¹H NMR (400 MHz, DMSO-d₆) δ 87.55 (d, *J*=8.2 Hz, 1H), 6.34 (d, *J*=12.1 Hz, 1H), 2.02 (s, 3H).

Lithium aluminum hydride (14.9 g, 382.0 mmol) and dry tetrahydrofuran (THF) (100.0 mL) were added to a reaction flask, and cooled to 0°C with stirring. A solution of **13c** (26.0 g, 152.8 mmol) in THF (400.0 mL) was added. The reaction solution was stirred at room temperature for 3 h, and then quenched with a small amount of saturated Na₂SO₄ aqueous solution. The solution subjected to quenching was filtered. The filtrate was concentrated, and water was added. The filtrate mixture was extracted with ethyl acetate. The resulting organic phase was washed with a saturated NaCl aqueous solution, dried over anhydrous sodium sulfate, filtered, and concentrated to afford a pale-yellow liquid product **13d** (22.1 g, yield: 92.9%, HPLC: 94%). LCMS: [M+H]⁺ 156.11 (theoretical value: 155.07); ¹H NMR (600 MHz, DMSO-d₆) δ 66.90 (d, *J*=9.0 Hz, 1H), 4.98 (s, 2H), 4.95 (t, *J*=5.6 Hz, 1H), 4.32 (d, *J*=5.1 Hz, 2H), 2.05 (s, 3H).

**13d** (22.1 g, 141.0 mmol), DCM (450.0 mL), and manganese dioxide (59.0 g, 707.0 mmol) were added to a reaction flask. The reaction solution was stirred at room temperature for 16 h, filtered and washed with DCM. The filtrate was concentrated to afford **13e** (21.0 g, yield: 87%). LCMS: [M+H]⁺ 154.10 (theoretical value: 153.06); ¹H NMR (400 MHz, CDCl₃) δ 9.75 (s, 1H), 7.27 (d, *J*=8.3 Hz, 1H), 6.29 (d, *J*=11.5 Hz, 1H), 6.12 (s, 2H), 2.17 (s, 3H).

**13e** (21.0 g, 136.2 mmol), the tricyclic intermediate **5b** (36.0 g, 136.2 mmol), PPTS (13.8 g, 136.22 mmol), and toluene (600.0 mL) were added to a reaction flask. The reaction solution was heated to 110°C under stirring for reaction overnight, and then cooled to room temperature. MeOH (400.0 mL) was added. The mixture was stirred at room temperature for 2 h to precipitate a solid. The solid was collected by filtration to afford **13f** (38.5 g, yield: 72%, HPLC: 97%). LCMS: [M+H]⁺ 381.3 (theoretical value: 380.12); ¹H NMR (500 MHz, DMSO-d₆) δ 8.60 (s, 1H), 8.03 (d, *J*=8.3 Hz, 1H), 7.86 (d, *J*=10.9 Hz, 1H), 7.31 (s, 1H), 6.52 (s, 1H), 5.42 (s, 2H), 5.22 (s, 2H), 2.47 (s, 3H), 1.87 (ddt, *J*=17.2, 14.1, 7.1 Hz, 2H), 0.89 (t, *J*=7.3 Hz, 3H).

**13f** (30.0 g, 78.7 mmol), CH₃OH (450.0 mL), and H₂O (375.0 mL) were added to a reaction flask, and cooled to 0°C. 75% H₂SO₄ (375.0 mL) was added dropwise, and the temperature was maintained below 10°C. At 0°C, ferrous sulfate hydrate (22.0 g, 78.7 mmol) and 30% H₂O₂ (160.0 mL) were added under stirring. The reaction solution was further stirred at room temperature for 16 h, and then poured into ice water (6.0 L) to precipitate a solid. The solid was collected by filtration, washed with ACN, and dried to afford **13g** (18.2 g, yield: 56%, HPLC: 97%). LCMS: [M+H]⁺ 411.1, (theoretical value: 410.13); ¹H NMR (500 MHz, DMSO-d₆) δ 8.14 (d, *J*=8.2 Hz, 1H), 7.88 (d, *J*=10.7 Hz, 1H), 7.32 (s, 1H), 5.49 (s, 1H), 5.43 (s, 2H), 5.39 (s, 2H), 5.26 (s, 2H), 3.17 (s, 1H), 1.87 (qd, *J*=14.6, 14.2, 7.0 Hz, 3H), 1.24 (s, 2H), 0.89 (t, *J*=7.3 Hz, 3H).

**13g** (14.0 g, 34.0 mmol), sodium bicarbonate (11.4 g, 136.1 mmol), DCM (260.0 mL), DMSO (45.0 mL), and Dess-Martin periodinane (28.9 g, 67.9 mmol) were added to a reaction flask. The reaction solution was stirred at room temperature for 16 h, and then concentrated. Water was added to precipitate a solid. The solid was collected by filtration and dried to afford **13h** (21.3 g, yield: 77%). LCMS: [M+H]⁺ 409.34 (theoretical value: 408.11); ¹H NMR (600 MHz, CDCl₃) δ 11.00 (s, 1H), 8.90 (d, *J*=7.7 Hz, 1H), 7.95 (d, *J*=10.3 Hz, 1H), 7.31 (s, 1H), 6.56 (s, 1OH), 5.44 (s, 2H), 5.42 (s, 2H), 2.48 (s, 3H), 1.88 (m, 2H), 0.89 (t, *J*=7.4 Hz, 8H).

**13h** (5 g, 9.77 mmol), 2,2,2-trifluoroethylamine (3.97 g, 29.31 mmol, 3.19 mL), DCM (80 mL), and DMSO (20 mL) were added to a reaction flask and stirred at room temperature for 30 min. NaBH₃CN (1.84 g, 29.31 mmol) was added, and the mixture was further stirred at room temperature for 2 h and then concentrated. Then, a saturated NaHCO₃ aqueous solution was added to precipitate a solid. The solid was collected by filtration and purified by silica gel column chromatography to afford a pale-yellow solid product **13** (1.1 g, yield: 21.72%, HPLC: 95%). LCMS: [M+H]⁺ 492.22 (theoretical value: 491.44); ¹H NMR (500 MHz, DMSO-d₆) *δ* 8.28 (d, *J*=8.2 Hz, 1H), 7.87 (d, *J*=10.7 Hz, 1H), 7.32 (s, 1H), 6.53 (s, 1H), 5.44 (s, 2H), 5.36 (s, 2H), 4.40 (d, *J*=6.7 Hz, 2H), 3.44 (td, *J*=10.1, 7.0 Hz, 2H), 3.18 (p, *J*=6.9 Hz, 1H), 2.50 (s, 3H), 1.88 (dp, *J*=18.3, 7.1 Hz, 2H), 0.89 (t, *J*=7.3 Hz, 3H); ¹³C NMR (126 MHz, DMSO) *δ* 172.95, 161.26, 157.25, 152.95, 150.48, 149.07, 145.93, 141.14, 128.99, 127.86, 127.49, 127.27, 124.54, 119.53, 112.91, 112.74, 97.12, 72.84, 65.73, 50.50, 49.84, 49.60, 47.30, 30.73, 15.73, 8.22.

### Example 14: 7-(N-(S)-(1',1',1'-trifluoropropan-2'-yl)amino)methyl-10-methyl-11-fluorocamptothecin (14)

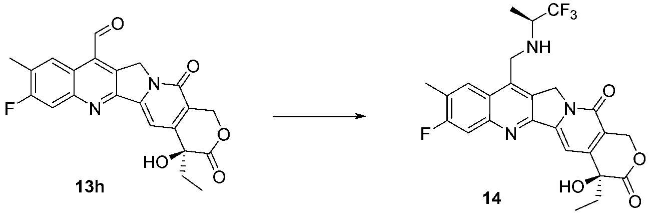

**13h** (5.0 g, 12.2 mmol), (S)-1,1,1-trifluoropropan-2-amine hydrochloride (5.5 g, 36.6 mmol), DMSO (45.0 mL), DCM (260.0 mL), and NaBH₃(CN) (2.3 g, 36.6 mmol) were added to a reaction flask. The reaction solution was stirred at room temperature for 3 h, and then concentrated. Then, H₂O (100.0 mL) was added to precipitate a solid. The solid was collected by filtration and purified by silica gel column chromatography to afford a yellow solid product **14** (2.1 g, yield: 65.2%, HPLC: 96%). LCMS: [M+H]⁺ 506.30 (theoretical value: 505.47); ¹H NMR (500 MHz, DMSO-d₆) δ 8.30 (d, *J*=8.3 Hz, 1H), 7.88 (d, *J*=10.7 Hz, 1H), 7.32 (s, 1H), 6.52 (s, 1H), 5.44 (s, 2H), 5.39 (s, 2H), 4.48 (dd, *J*=13.8, 6.1 Hz, 1H), 4.38 (dd, *J*=13.9, 7.7 Hz, 1H), 3.59-3.46 (m, 1H), 2.89 (q, *J*=7.2 Hz, 1H), 1.88 (dp, *J*=19.1, 7.1 Hz, 2H), 1.44-1.12 (m, 6H), 0.88 (t, *J*=7.3 Hz, 3H).

### Example 15: 7-(N-(R)-(1',1',1'-trifluoropropan-2'-yl)amino)methyl-10-methyl-11-fluorocamptothecin (15)

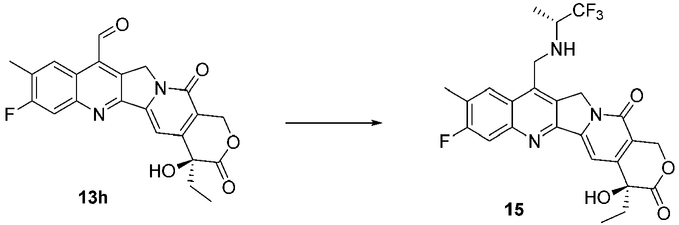

**13h** (0.6 g, 1.47 mmol), (R)-1,1,1-trifluoropropan-2-amine (639.87 mg, 4.40 mmol), DCM (15 mL), and DMSO (3 mL) were added to a reaction flask and stirred at room temperature for 1 h. NaBH₃CN (276.30 mg, 4.40 mmol) was added, and the mixture was further stirred at room temperature for 2 h and then concentrated. Then, a saturated aqueous sodium bicarbonate solution was added to precipitate a solid. The solid was collected by filtration and purified by silica gel column chromatography to afford a pale-yellow solid product **15** (0.2 g, yield: 26%, HPLC: 96%). LCMS: [M+H]⁺ 506.21 (theoretical value: 505.47); ¹H NMR (500 MHz, DMSO-d₆) δ 8.34-8.26 (m, 1H), 7.88 (d, J=10.8 Hz, 1H), 7.32 (s, 1H), 6.52 (s, 1H), 5.44 (s, 2H), 5.39 (d, J=2.4 Hz, 2H), 4.48 (dd, *J*=13.8, 6.1 Hz, 1H), 4.37 (dd, *J*=13.8, 7.8 Hz, 1H), 3.53 (h, *J*=7.3 Hz, 1H), 2.89 (q, *J*=7.2 Hz, 1H), 1.87 (dh, *J*=21.3, 7.2 Hz, 2H), 1.27 (d, *J*=6.8 Hz, 3H), 0.88 (t, *J*=7.3 Hz, 3H).

### Example 16: 7-(N-(2'-trifluoromethylpropan-2'-yl)amino)methyl-10-methyl-11-fluorocamptothecin (16)

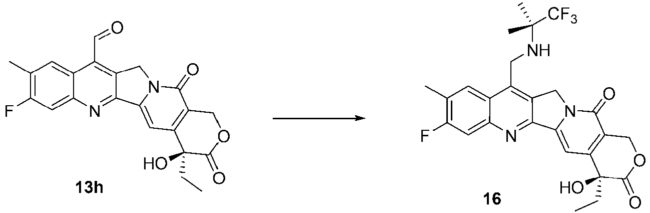

**13h** (100 mg, 244.87 µmol), 2-trifluoromethylpropan-2-amine hydrochloride (120.16 mg, 734.61 µmol), DMSO (1.0 mL), and DCM (5.0 mL) were added to a reaction flask and stirred at room temperature for 30 min. NaBH₃CN (46.16 mg, 734.61 µmol) was added, and the mixture was further stirred at room temperature overnight. Then, a saturated aqueous sodium bicarbonate solution was added to precipitate a solid. The solid was collected by filtration and purified by silica gel column chromatography to afford a pale-yellow solid product **16** (40 mg, yield: 32%, HPLC: 95%). LCMS: [M+H]⁺ 520.20 (theoretical value: 519.18); ¹H NMR (500 MHz, DMSO-*d*₆) δ 8.25 (d, *J*=8.4 Hz, 1H), 7.90 (d, *J*=10.9 Hz, 1H), 7.33 (s, 1H), 6.52 (s, 1H), 5.43 (d, *J*=7.8 Hz, 4H), 4.38 (d, *J*=7.4 Hz, 2H), 2.94 (t, *J*=7.4 Hz, 1H), 1.88 (dq, *J*=14.3, 7.0 Hz, 2H), 1.38 (s, 6H), 0.88 (t, *J*=7.3 Hz, 3H).

### Example 17: 7-(N-(2',2',2'-trifluoroethyl)amino)ethylcamptothecin (17)

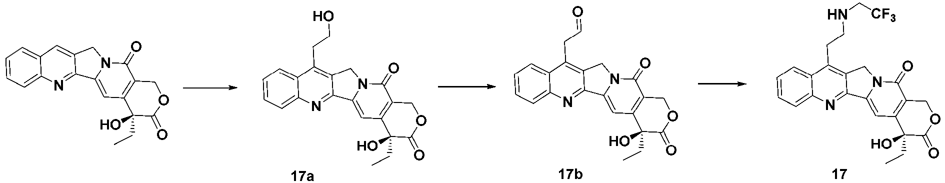

At 0°C, camptothecin (1 g, 2.86 mmol) and FeSO₄·7H₂O (795.78 mg, 2.86 mmol) were added to a 100 mL round-bottom flask and dissolved in H₂O (32.5 mL). H₂SO₄ (17.5 mL), 1,3-propanediol (10.59 g, 137.39 mmol, 10.06 mL), and 35% hydrogen peroxide (97.36 mg, 2.86 mmol, 87.71 µL) were carefully added. The mixture was stirred for dissolution and further reacted at 0°C for 3 h. The reaction solution was poured into 1 L of ice water to precipitate a solid. The solid was collected by filtration, dissolved in methanol, purified by silica gel column chromatography, and concentrated to afford a pale-yellow powdery solid **17a** (345 mg, yield: 29.11%). LCMS: [M+1]⁺ 393.43 (calcd. 392.41).

At 25°C, **17a** (345 mg, 0.833 mmol) prepared above was dissolved in DMSO (20 mL) and DCM (6 mL) in a 250 mL round-bottom flask. At 0°C, Dess-Martin periodinane (706 mg, 1.666 mmol) was added. The mixture reacted at 25°C for 3 h. Water (100 mL) was added. The reaction solution was extracted with DCM (100 mL × 3). The organic phases were combined, dried over anhydrous magnesium sulfate, and filtered. The filtrate was concentrated under reduced pressure to afford a brown viscous solid **17b** (291 mg, yield: 85%). LCMS: [M+H]⁺ 391.23 (calcd. 390.40).

**17b** (200 mg, 0.512 mmol) prepared above was dissolved in EtOH (5 mL). 2,2,2-trifluoroethylamine hydrochloride (162.64 mg, 1.2 mmol, HCl salt) and sodium acetate (100.8 mg, 1.2 mmol) were added, and the mixture was stirred at room temperature for 6 h. Acetic acid (15 mL) and sodium cyanoborohydride (94.5 mg, 1.5 mmol) were added, and the mixture reacted at room temperature for 2 h. Water was added, and the reaction solution was extracted with DCM (100 mL × 4). The organic phases were combined, dried over anhydrous magnesium sulfate, and filtered. The filtrate was concentrated under reduced pressure, and then purified by reversed-phase C18 column chromatography (ACN/0.1% trifluoroacetic acid (TFA) in water) to afford a pale-yellow powdery solid product **17** (18 mg, yield: 7.4%). LCMS: [M+1]⁺ 474.38 (calcd. 473.45); ¹H NMR (500 MHz, DMSO-d₆) δ 8.32 (dd, *J*=8.5, 1.3 Hz, 1H), 8.22 (dd, *J*=8.5, 1.2 Hz, 1H), 7.90 (ddd, *J*=8.3, 6.8, 1.3 Hz, 1H), 7.80 (ddd, *J*=8.3, 6.8, 1.3 Hz, 1H), 7.36 (s, 1H), 6.55 (s, 1H), 5.46 (s, 2H), 5.38 (s, 2H), 3.51 (s, 2H), 1.89 (ddt, *J*=17.8, 14.1, 7.2 Hz, 2H), 0.88 (q, *J*=8.6, 7.9 Hz, 3H).

### Example 18: 7-(N-(S)-(1',1',1'-trifluoropropan-2'-yl)amino)ethylcamptothecin (18)

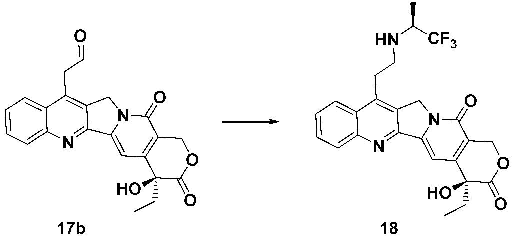

**17b** (200 mg, 0.512 mmol) was dissolved in EtOH (5 mL). (S)-1,1,1-trifluoropropan-2-amine (179.4 mg, 1.2 mmol, HCl salt) and sodium acetate (100.8 mg, 1.2 mmol) were added, and the mixture was stirred at room temperature for 6 h. Acetic acid (15 mL) and sodium cyanoborohydride (94.5 mg, 1.5 mmol) were added, and the mixture reacted at room temperature for 2 h. Water was added, and the reaction solution was extracted with DCM (100 mL × 4). The organic phases were combined, dried over anhydrous magnesium sulfate, and filtered. The filtrate was concentrated under reduced pressure, and purified by reversed-phase C18 column chromatography (ACN/0.1 % TFA in water). A collected fraction was lyophilized to afford a pale-yellow powdery solid product **18** (12 mg, yield: 5%). LCMS: [M+1]⁺ 488.68 (calcd. 487.48); ¹H NMR (500 MHz, DMSO-d₆) δ 8.32 (dd, *J*=8.5, 1.4 Hz, 1H), 8.20 (dd, *J*=8.5, 1.2 Hz, 1H), 7.88 (ddd, *J*=8.3, 6.8, 1.3 Hz, 1H), 7.85-7.72 (m, 1H), 7.35 (s, 1H), 6.62-6.46 (m, 1H), 5.46 (s, 2H), 5.38 (d, *J*=2.1 Hz, 2H), 3.13 (d, *J*=32.0 Hz, 2H), 1.94-1.84 (m, 2H), 1.27-1.21 (m, 3H), 0.89 (t, *J*=7.3 Hz, 3H).

### Example 19: 7-(N-(R)-(1',1',1'-trifluoropropan-2'-yl)amino)ethylcamptothecin (19)

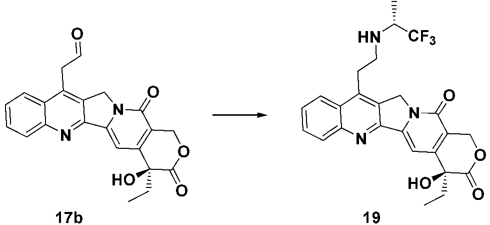

**17b** (200 mg, 0.512 mmol) was dissolved in EtOH (5 mL). (R)-1,1,1-trifluoro-2-propanamine (179.4 mg, 1.2 mmol, HCl salt) and sodium acetate (100.8 mg, 1.2 mmol) were added, and the mixture was stirred at room temperature for 6 h. Acetic acid (15 mL) and sodium cyanoborohydride (94.5 mg, 1.5 mmol) were added, and the mixture reacted at room temperature for 2 h. Water was added, and the reaction solution was extracted with DCM (100 mL × 4). The organic phases were combined, dried over anhydrous magnesium sulfate, and filtered. The filtrate was concentrated under reduced pressure, and purified by reversed-phase C18 column chromatography (ACN/0.1% TFA in water). A collected fraction was lyophilized to afford a pale-yellow powdery solid product **19** (8 mg, yield: 3%). LCMS: [M+1]⁺ 488.75 (calcd. 487.48); ¹H NMR (500 MHz, DMSO-d₆) δ 8.32 (d, *J*=8.4 Hz, 1H), 8.20 (d, *J*=8.4 Hz, 1H), 7.88 (t, *J*=7.6 Hz, 1H), 7.77 (t, *J*=7.7 Hz, 1H), 7.35 (s, 1H), 6.54 (s, 1H), 5.42 (d, *J*=37.4 Hz, 4H), 3.41-3.38 (m, 2H), 3.20-3.00 (m, 2H), 1.89 (dt, *J*=18.1, 7.0 Hz, 2H), 1.33-1.17 (m, 4H), 0.89 (t, *J*=7.3 Hz, 3H).

### Example 20: 7-(N-(2'-trifluoromethylpropan-2'-yl)amino)ethylcamptothecin (20)

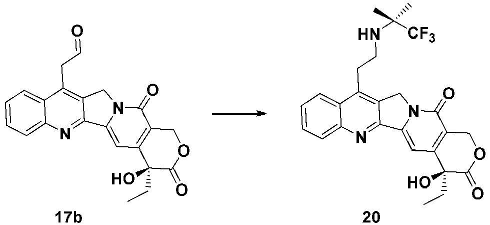

**17b** (200 mg, 0.512 mmol) was dissolved in EtOH (5 mL). 1,1,1-trifluoro-2-methyl-2-propanamine (196.2 mg, 1.2 mmol, HCl salt) and sodium acetate (100.8 mg, 1.2 mmol) were added, and the mixture was stirred at room temperature for 6 h. Acetic acid (15 mL) and sodium cyanoborohydride (94.5 mg, 1.5 mmol) were added, and the mixture was conducted at room temperature for 2 h. Water was added, and the reaction solution was extracted with DCM (100 mL × 4). The organic phases were combined, dried over anhydrous magnesium sulfate, and filtered. The filtrate was concentrated under reduced pressure, and purified by reversed-phase C18 column chromatography (ACN/0.1% TFA in water). A collected fraction was lyophilized to afford a pale-yellow powdery solid product **20** (10 mg, yield: 4%). LCMS: [M+1]⁺ 502.45 (calcd. 501.51); ¹H NMR (500 MHz, DMSO-d₆) δ 8.32 (d, *J*=8.6 Hz, 1H), 8.20 (dd, *J*=8.5, 1.2 Hz, 1H), 7.92-7.84 (m, 1H), 7.79-7.68 (m, 1H), 7.36 (s, 1H), 5.69 (s, 1H), 5.46 (d, *J*=13.2 Hz, 4H), 3.60-3.55 (m, 2H), 3.19-3.02 (m, 2H), 1.89 (dt, *J*=16.6, 7.1 Hz, 2H), 1.29-1.12 (m, 6H), 0.90 (t, *J*=7.3 Hz, 3H).

### Example 21: 7-(N-(2',2',2'-trifluoroethyl)amino)ethyl-10-methyl-11-fluorocamptothecin (21)

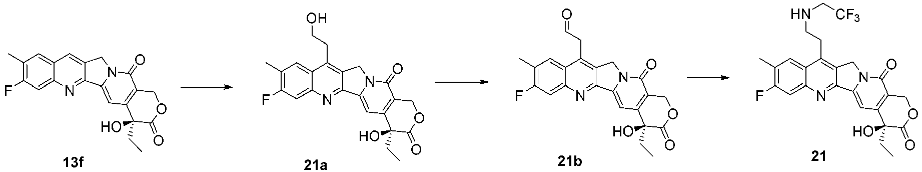

25 mL of deionized water was added to a 500 mL three-necked flask. 25 mL of 75% sulfuric acid was slowly added dropwise under ice-bath cooling. Then, 25 mL of 1,3-propanediol, **13f** (5.0 g, 13.1 mmol), and ferrous sulfate heptahydrate (7.2 mg, 26.2 mmol) were added. The mixture was allowed to become clear and then cooled to 0°C. A 30% aqueous H₂O₂ solution (17 g, 260 mmol) was added dropwise, and the temperature was maintained below 15°C. The reaction solution was stirred for 30 min and then poured into water to precipitate a solid. The solid was collected by filtration, oven-dried, and purified by silica gel column chromatography to afford a product **21a** (2.3 g, yield: 33.4%). LCMS: [M+1]⁺ 425.35 (calcd. 424.41).

**21a** (300 mg, 0.7 mmol) was added to a 100 mL three-necked flask, and a DCM/DMSO (1:1, 30 mL) mixed solution was then added for dissolution. Dess-Martin periodinane (742 mg, 1.75 mmol) was then added. The mixture reacted at room temperature for 10 min. The reaction solution was poured into ice water and extracted with DCM (50 mL × 3). The organic phases were combined, washed with saturated brine, dried over anhydrous sodium sulfate, and then concentrated under reduced pressure to afford a crude product **21b** (280 mg, yield: 94%), which was used directly in the next step without further purification. LCMS: [M+1]⁺ 423.35 (calcd. 422.41).

**21b** (280 mg, 0.66 mmol) was dissolved in EtOH (5 mL) in a 100 mL single-necked flask. Under stirring, 2,2,2-trifluoroethylamine hydrochloride (1.43 g, 10.6 mmol) and sodium acetate (869.5 mg, 10.6 mmol) were added. The mixture reacted at room temperature for 30 min. Sodium cyanoborohydride (999 mg, 15.9 mmol) was added, and 15 mL of glacial acetic acid was then added. The resulting mixture was stirred at room temperature for 1 h. Water (10 mL) was added, and the reaction solution was extracted with DCM (20 mL × 3). The organic phases were combined, washed with saturated brine, dried over anhydrous sodium sulfate, concentrated, purified by reversed-phase preparative HPLC, and lyophilized to afford a yellow solid product **21** (9 mg, yield: 3%). LCMS: [M+1]⁺ 506.31 (calcd. 505.47); ¹H NMR (500 MHz, DMSO-d₆) δ 8.22 (d, *J*=7.2 Hz, 1H), 7.88 (d, *J*=10.5 Hz, 1H), 7.31 (s, 1H), 6.53 (s, 1H), 5.44 (s, 2H), 5.32 (s, 2H), 3.14 (s, 7H), 2.51 (s, 3H), 2.00-1.72 (m, 2H), 0.88 (t, *J*=6.5 Hz, 3H).

### Example 22: 7-(N-(S)-(1',1',1'-trifluoropropan-2'-yl)amino)ethyl-10-methyl-11-fluorocamptothecin (22)

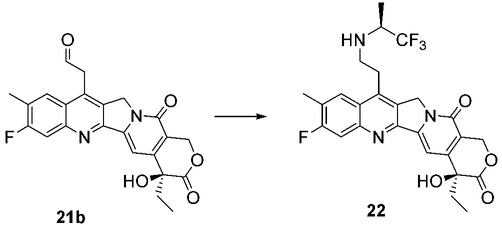

**21b** (200 mg, 0.47 mmol) was dissolved in EtOH (2 mL) in a 100 mL single-necked flask. Under stirring, (S)-1,1,1-trifluoroisopropylamine hydrochloride (702.8 mg, 4.7 mmol) and sodium acetate (385.5 m g, 4.7 mmol) were added. The mixture reacted at room temperature for 30 min. Sodium cyanoborohydride (443 mg, 7.05 mmol) was added, and 6 mL of glacial acetic acid was then added. The resulting mixture was stirred at room temperature for 1 h. Water (15 mL) was added, and the reaction solution was extracted with DCM (20 mL × 3). The organic phases were combined, washed with saturated brine, dried over anhydrous sodium sulfate, concentrated, purified by reversed-phase preparative HPLC, and lyophilized to afford a yellow solid product **22** (18 mg, yield: 7.3%). LCMS: [M+1]⁺ 520.51 (calcd. 519.5); ¹H NMR (500 MHz, DMSO- d₆) δ 8.16 (d, *J*=8.1 Hz, 1H), 7.81 (d, *J*=10.7 Hz, 1H), 7.28 (s, 1H), 6.52 (s, 1H), 5.43 (s, 2H), 5.23 (s, 2H), 3.94-3.65 (m, 2H), 3.40-3.30 (m, 2H), 3.10 (d, *J*=32.8 Hz, 2H), 2.48 (s, 3H), 1.98-1.77 (m, 2H), 1.24 (d, *J*=6.1 Hz, 2H), 0.89 (t, *J*=7.3 Hz, 3H).

### Example 23: 7-(N-(R)-(1',1',1'-trifluoropropan-2'-yl)amino)ethyl-10-methyl-11-fluorocamptothecin (23)

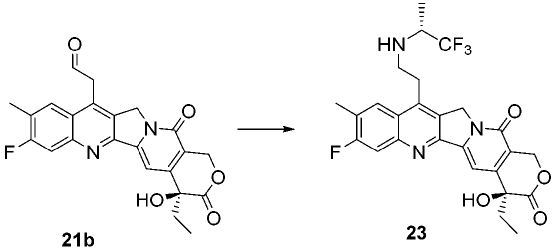

**21b** (200 mg, 0.47 mmol) was dissolved in EtOH (2 mL) in a 100 mL single-necked flask. Under stirring, (R)-1,1,1-trifluoroisopropylamine hydrochloride (702.8 mg, 4.7 mmol) and sodium acetate (385.5 mg, 4.7 mmol) were added. The mixture reacted at room temperature for 30 min. Sodium cyanoborohydride (443 mg, 7.05 mmol) and 6 mL of glacial acetic acid were added. The resulting mixture was stirred at room temperature for 1 h. Water (15 mL) was added, and the reaction solution was extracted with DCM (20 mL × 3). The organic phases were combined, washed with saturated brine, dried over anhydrous sodium sulfate, concentrated, purified by reversed-phase preparative HPLC, and lyophilized to afford a yellow solid product **23** (14 mg, yield: 5.7%). LCMS: [M+1]⁺ 520.51 (calcd. 519.5); ¹H NMR (500 MHz, DMSO-d₆) δ 8.20 (d, *J*=8.1 Hz, 1H), 7.86 (d, *J*=10.8 Hz, 1H), 7.30 (s, 1H), 6.53 (s, 1H), 5.44 (s, 2H), 5.29 (s, 2H), 3.89-3.46 (m, 4H), 3.11 (d, *J*=35.2 Hz, 2H), 2.51 (s, 3H), 2.03-1.76 (m, 2H), 1.24 (d, *J*=5.4 Hz, 3H), 0.88 (t, *J*=7.2 Hz, 3H).

### Example 24: 7-(N-(2'-trifluoromethylpropan-2'-yl)amino)ethyl-10-methyl-11-fluorocamptothecin (24)

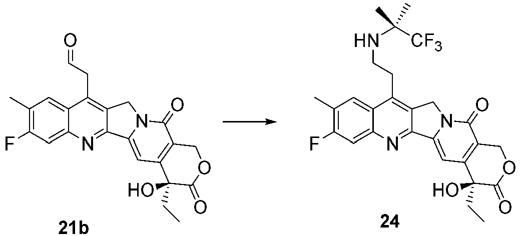

**21b** (200 mg, 0.47 mmol) was dissolved in EtOH (2 mL) in a 100 mL single-necked flask. Under stirring, 2,2,2-trifluoro-1,1-dimethylethylamine hydrochloride (768.7 mg, 4.7 mmol) and sodium acetate (385.5 mg, 4.7 mmol) were added. The mixture reacted at room temperature for 30 min. Sodium cyanoborohydride (443 mg, 7.05 mmol) was added, and 6 mL of glacial acetic acid was then added. The resulting mixture was stirred at room temperature for 1 h. Water (15 mL) was added, and the reaction solution was extracted with DCM (20 mL × 3). The organic phases were combined, washed with saturated brine, dried over anhydrous sodium sulfate, concentrated, purified by reversed-phase preparative HPLC, and lyophilized to afford a yellow solid product **24** (19 mg, yield: 7.6%). LCMS: [M+1]⁺ 534.3 (calcd. 533.52); ¹H NMR (500 MHz, DMSO-d₆) δ 8.15 (d, *J*=7.8 Hz, 1H), 7.79 (d, *J*=10.7 Hz, 1H), 7.28 (s, 1H), 6.51 (s, 1H), 5.43 (s, 2H), 5.22 (s, 2H), 3.30-3.15 (m, 2H), 3.00-2.96 (m, 2H), 2.48 (s, 3H), 2.00-1.80 (m, 2H), 1.09 (s, 6H), 0.89 (t, *J*=7.0 Hz, 3H).

### Example 25: 7-(N-(2',2',2'-trifluoroethyl)amino)ethyl-10,11-difluorocamptothecin (25)

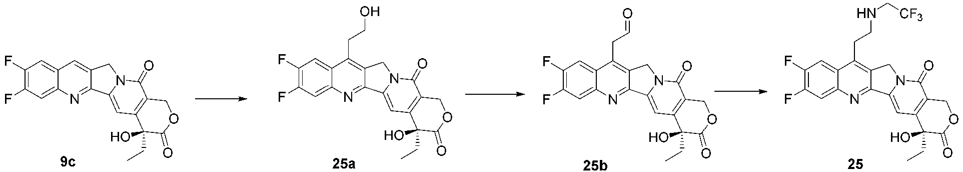

**9c** (3 g, 7.37 mmol), H₂O (75 mL), and 1,3-propanediol (62.07 g, 626.45 mmol, 58.95 mL) were added to a 500 mL three-necked flask. H₂SO₄ (75%) (36.14 g, 368.50 mmol, 75 mL) was then added. At 0°C, ferrous sulfate heptahydrate (2.34 g, 8.40 mmol) was added. Then, a 30% aqueous H₂O₂ solution (215.59 mg, 6.34 mmol, 194.23 µL) was added dropwise. The reaction solution was stirred at 0°C for 4 h, then poured into 2 L of ice water, and allowed to stand for 16 h, such that a solid was precipitated. The solid was collected by filtration and dried to afford **25a** (1.8 g, yield: 54.11%). LCMS: [M+1]⁺ 429.31 (calcd. 428.39).

**25a** (1 g, 2.22 mmol), DMSO (20 mL), and DCM (14 mL) were added to a 100 mL three-necked flask. Dess-Martin periodinane (1.88 g, 4.44 mmol) was then added. The reaction solution was stirred at 25°C for 4 h, poured into ice water, and extracted with DCM (50 mL × 3). The organic phases were combined, washed with saturated brine, dried over anhydrous sodium sulfate, and then concentrated under reduced pressure to afford a crude product **25b** (320 mg, yield: 33.8%), which was used directly in the next step without further purification. LCMS: [M+1]⁺ 427.16 (calcd. 426.38).

**25b** (50 mg, 111.27 µmol), DMSO (6.00 mL), and DCM (4.00 mL) were added to a 100 mL single-necked flask. Then 2,2,2-trifluoroethylamine (40.74 mg, 333.81 µmol, 32.72 µL) was added. The mixed solution was stirred at 25°C for 1 h. NaBH₃CN (13.98 mg, 222.54 µmol) and acetic acid (33.41 mg, 556.35 µmol) were added. The reaction solution was stirred at 25°C for 16 h, concentrated, purified by a C18 Spherical 20-35 µm 100 A 120 g reversed-phase column, and lyophilized to afford a product **25** (19.41 mg, yield: 32.76%). LCMS: [M+1]⁺ 510.15 (calcd. 509.43); ¹H NMR (600 MHz, DMSO-d₆) δ 8.47-8.29 (m, 1H), 8.23-8.04 (m, 1H), 7.29 (s, 1H), 6.53 (s, 1H), 5.43 (s, 2H), 5.29 (s, 2H), 2.97 (s, 2H), 2.71 (s, 1H), 1.87 (dq, *J*=14.1, 7.0 Hz, 2H), 0.88 (t, *J*=7.1 Hz, 3H).

### Example 26: 7-(N-(R)-(1',1',1'-trifluoropropan-2'-yl)amino)ethyl-10,11-difluorocamptothecin (26)

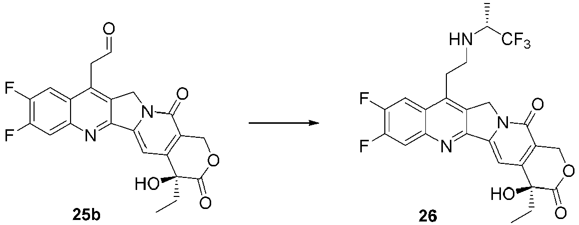

**25b** (15 mg, 33.38 µmol), 1,4-dioxane (1 mL), and DCM (1 mL) were added to a 10 mL reaction flask. A solution of (2R)-1,1,1-trifluoro-2-propanamine (4.99 mg, 33.38 µmol) and DIPEA (8.63 mg, 66.76 µmol, 11.63 µL) in DCM (1 mL) was added to the above reaction solution and then stirred for 10 min. NaBH₃CN (4.20 mg, 66.76 µmol) and acetic acid (4.01 mg, 66.76 µmol) were added. The reaction solution was stirred at 25°C for 2 h and concentrated. The concentrate was dissolved in 1 mL of DMF, purified by HPLC preparative chromatography (monitored at wavelengths of 254 nm and 214 nm) using a C18 Spherical 20-35 µm 100 A 20 g reversed-phase column with ACN (HPLC grade) as a mobile phase B2 and a 0.1% TFA aqueous solution as a mobile phase A2, and lyophilized to afford a product **26** (5 mg, yield: 27.41%). LCMS: [M+1]⁺ 524.23 (calcd. 523.46); ¹H NMR (500 MHz, DMSO-d₆) δ 8.46-8.37 (m, 1H), 8.25-8.16 (m, 1H), 7.31 (s, 1H), 6.54 (s, 1H), 5.45 (s, 2H), 5.37 (s, 2H), 5.33 (s, 1H), 2.19 (t, *J*=7.3 Hz, 4H), 1.12 (s, 3H), 0.85 (s, 3H).

### Example 27: 7-(N-(S)-(1',1',1'-trifluoropropan-2'-yl)amino)ethyl-10,11-difluorocamptothecin (27)

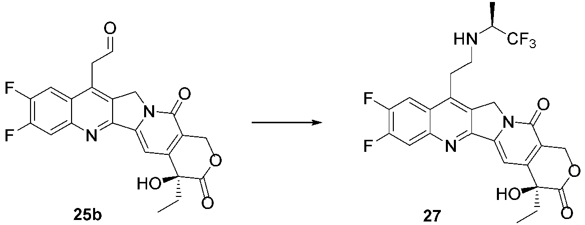

**25b** (50 mg, 111.27 µmol), DMSO (6.02 mL), and DCM (4.01 mL) were added to a 100 mL single-necked flask. Then, (2S)-1,1,1-trifluoro-2-propanamine (15.14 mg, 111.27 µmol, 13.19 µL) was added. The mixed solution was stirred at 25°C for 1 h. NaBH₃CN (13.98 mg, 222.54 µmol) and acetic acid (33.41 mg, 556.35 µmol) were added. The reaction solution was stirred at 25°C for 16 h, concentrated, purified by HPLC preparative chromatography (monitored at wavelengths of 254 nm and 214 nm) using a C18 Spherical 20-35 µm 100 A 120 g reversed-phase column with ACN (HPLC grade) as a mobile phase B2 and a 0.1% TFA aqueous solution as a mobile phase A2, and lyophilized to afford a product **27** (10.45 mg, yield: 17.94%). LCMS: [M+1]⁺ 524.33 (calcd. 523.46); ¹H NMR (600 MHz, DMSO-d₆) δ 8.37 (s, 1H), 8.20 (s, 1H), 7.32 (s, 1H), 6.54 (s, 1H), 5.44 (s, 2H), 5.35 (s, 2H), 3.08 (d, *J*=49.6 Hz, 2H), 1.87 (dd, *J*=14.2, 7.2 Hz, 2H), 1.21 (s, 3H), 0.88 (t, *J*=7.1 Hz, 3H).

### Example 28: 7-(N-(S)-(1',1',1'-trifluoropropan-2'-yl)amino)ethyl-10,11-methylenedioxycamptothecin (28)

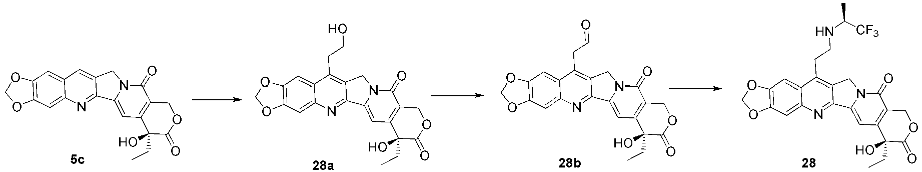

At 0°C, water (361.65 mL), **5c** (5 g, 12.71 mmol), FeSO₄·7H₂O (11.31 g, 40.67 mmol), and 1,3-propanediol (28.9 g, 381.3 mmol) were successively added to a 1,000 mL round-bottom flask and thoroughly stirred. Concentrated sulfuric acid (180.82 mL) was slowly added dropwise, and then 30% hydrogen peroxide (5.82 g, 171.45 mmol) was then added dropwise. The reaction solution reacted at 0°C for 3 h, and then poured into 3 L of ice water to precipitate a solid. The solid was collected by filtration and purified by silica gel column chromatography to afford a pale-yellow powdery solid **28a** as an intermediate (1.67 g, yield: 28.53%). MS: [M+1]⁺ 437.32 (calcd. 436.42); ¹H NMR (500 MHz, DMSO-d₆) δ 7.64 (s, 1H), 7.52 (s, 1H), 7.28 (s, 1H), 6.54 (s, 1H), 6.33 (s, 2H), 5.47 (s, 2H), 5.25 (s, 2H), 3.82 (d, *J*=6.4 Hz, 1H), 3.29 (t, *J*=5.9 Hz, 2H), 1.92 (p, *J*=7.8, 7.4 Hz, 2H), 1.75 (d, *J*=6.4 Hz, 1H), 0.94 (t, *J*=7.3 Hz, 3H).

**28a** (800 mg, 1.83 mmol), DMSO (50 mL), and DCM (35 mL) were added to a reaction flask. Dess-Martin periodinane (1.55 g, 3.66 mmol) was added. The reaction solution was stirred at room temperature for 1 hour. Water (100 mL) was added, and the reaction solution was extracted with DCM (100 mL × 3). The organic phases were combined, dried over anhydrous magnesium sulfate, and filtered. The filtrate was concentrated under reduced pressure to afford a brown viscous solid **28b** as an intermediate (796.31 mg, yield: 90%), which was used directly in the next step without further purification. LCMS: [M+H]⁺ 435.33 (calcd. 434.40).

**28b** (950 mg, 1.4 mmol), DCM (10 mL), DMSO (4 mL), and (S)-1,1,1-trifluoropropan-2-amine (780 mg, 7 mmol) were successively added to a 100 mL reaction flask at room temperature, and stirred at room temperature for 60 min. Then, sodium cyanoborohydride (130 mg, 2.1 mmol) was added. The reaction solution was further stirred for 2 h, concentrated, and purified by silica gel column chromatography to afford a product **28** (600 mg, yield: 80%). LCMS: [M+1]⁺ 532.03 (calcd. 531.49); ¹H NMR (600 MHz, DMSO-d₆) δ 7.55 (s, 1H), 7.41 (s, 1H), 7.20 (s, 1H), 6.48 (s, 1H), 6.27 (d, *J*=3.4 Hz, 2H), 5.54-5.33 (m, 2H), 5.09 (s, 2H), 3.34 (s, 2H), 3.23-3.06 (m, 2H), 2.90 (m, 2H), 1.95-1.80 (m, 2H), 1.14 (d, *J*=6.7 Hz, 3H), 0.94-0.83 (m, 3H); ¹³C NMR (150 MHz, DMSO-d₆) δ 172.98, 157.26, 151.20, 150.54, 149.62, 149.29, 147.47, 146.78, 140.98, 128.35, 127.78 (d, J=280 Hz), 124.84, 118.41, 105.83, 103.05, 99.83, 96.32, 72.86, 65.73, 54.45, 50.29, 47.31, 31.28, 30.79, 14.68, 8.24.

### Example 29: 7-(N-(R)-(1',1',1'-trifluoropropan-2'-yl)amino)ethyl-10,11-methylenedioxycamptothecin (29)

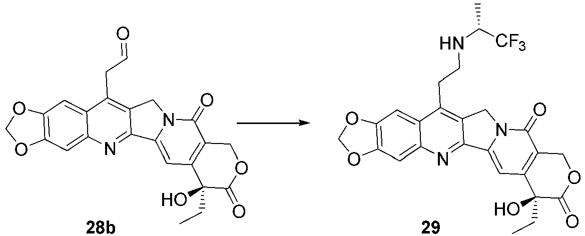

**28b** (30 mg, 69.06 µmol), DCM (2 mL), DMSO (2 mL), and (R)-1,1,1-trifluoro-2-propanamine (78.1 mg, 0.69 mmol) were successively added to a 10 mL single-necked eggplant-shaped flask at 25°C, and stirred at room temperature for 30 min. Then, sodium cyanoborohydride (13 mg, 207.2 µmol) was added. The reaction solution was further stirred for 2 h, concentrated, and purified by silica gel column chromatography to afford a brownish-yellow solid product **29** (10 mg, yield: 27.2%). LCMS: [M+1]⁺ 532.53 (calcd. 531.49); ¹H NMR (500 MHz, DMSO-d₆) δ 7.68 (s, 1H), 7.55 (s, 1H), 7.26 (s, 1H), 6.32 (s, 2H), 5.44 (d, *J*=1.3 Hz, 2H), 5.31 (d, *J*=3.0 Hz, 2H), 3.47 (d, *J*=16.4 Hz, 1H), 3.37-3.24 (m, 2H), 3.19 (s, 1H), 1.87 (dp, *J*=19.0, 7.2 Hz, 2H), 1.36 (d, *J*=6.8 Hz, 3H), 0.89 (q, *J*=7.9, 7.4 Hz, 3H), 0.87 (s, 1H).

### Test Example 1: Inhibitory activity against tumor cell growth

Human esophageal cancer cells OE33, human breast cancer cells MDA-MB-231, human gastric cancer cells NCI-N87, human lung cancer cells NCI-H1975, and human breast cancer cells SK-BR-3 were cultured in RPMI1640 (Cellmax) containing 10% fetal bovine serum (Cellmax). Tumor cells in the exponential growth phase were diluted with the medium to 1 × 10⁵ cells/mL to afford a cell suspension. The cell suspension was added to a 96-well cell culture plate at 100 µL/well, and cultured in a 37°C and 5% CO₂ incubator overnight. On the next day, a test compound was diluted with the medium to concentrations of 10,000 nM, 2,000 nM, 400 nM, 80 nM, 16 nM, 3.2 nM, 0.64 nM, and 0.13 nM. Compound dilutions were added to the 96-well cell culture plate at 2 µL/well, with three replicate wells for each concentration. For negative control and blank control groups without the test compound, a diluent was added at 2 µL/well. After sample addition, the 96-well cell culture plate was placed back into a 37°C and 5% CO₂ incubator for further incubation of cells for 72 h. After the incubation was completed, the cell culture plate was taken out, the medium in each well was discarded using a pipette, and then 100 µL of medium containing 10% CCK-8 was added to each well for further incubation of cells at 37°C for 3 h. After the incubation was completed, the culture plate was taken out and placed in a microplate reader while being protected from light. The absorbance was measured with a reference wavelength of 630 nm and a detection wavelength of 450 nm. Based on absorbance values, IC₅₀ values were calculated using four-parameter logistic regression in GraphPad (Table 1). Dxd was used as a positive control drug, with a structural formula

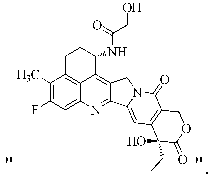

The exemplary compounds of the present disclosure exhibited excellent inhibitory activity against cancer cells such as OE33, MDA-MB-231, NCI-N87, NCI-H1975, and SK-BR-3, with IC₅₀ values all less than 500 nM. The IC₅₀ values of some compounds against these cancer cells were below 100 nM, 50 nM, or 10 nM, or even below 1 nM. For IC₅₀ values, "++++" indicates IC₅₀ ≤ 10 nM, "+++" indicates 10 nM < IC₅₀ ≤ 100 nM, "++" indicates 100 nM < IC₅₀ ≤ 500 nM, and "+" indicates 500 nM < IC₅₀ ≤ 1,000 nM.

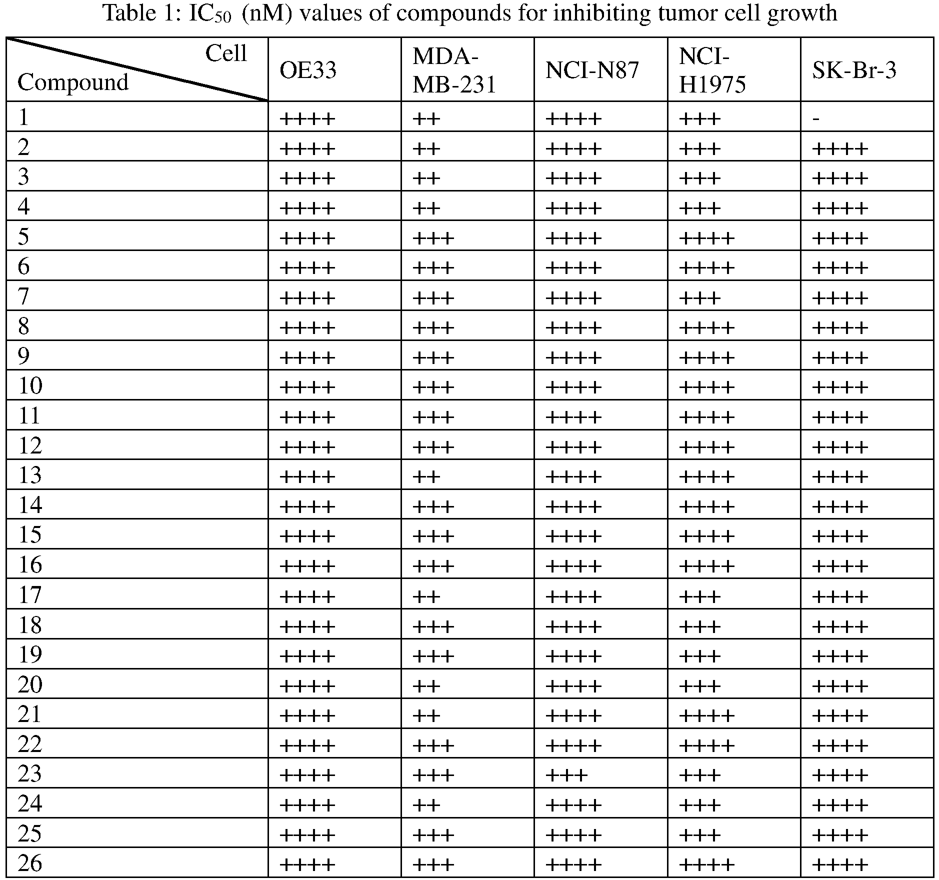

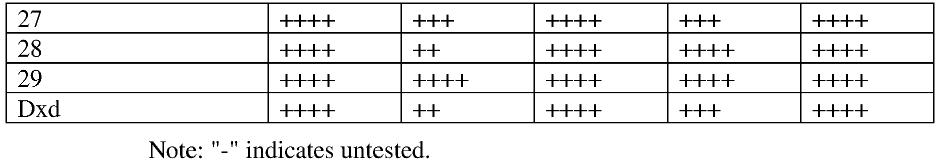

The specific exemplary embodiments of the present disclosure have been presented for illustrative and exemplary purposes. These descriptions are not intended to limit the present disclosure to the precise forms disclosed, and obviously, many modifications and variations may be made in light of the above teachings. The exemplary embodiments are selected and described to explain the specific principle of the present disclosure and a practical application thereof, such that those skilled in the art can make and utilize various different exemplary embodiments of the present disclosure and various alternatives and modifications thereof. The scope of the present disclosure is intended to be defined by the claims and equivalents thereof.

## Claims

1. A compound of Formula (I), and a pharmaceutically acceptable salt and a stereoisomer thereof: 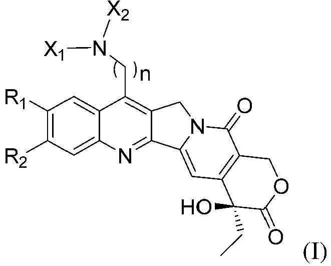 wherein,
R₁ and R₂ are each independently selected from hydrogen, fluorine and methyl, or
R₁ and R₂ together with the carbon atoms to which they are attached form 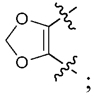
X₁ is selected from hydrogen and C₁-C₃ alkyl;
X₂ is selected from substituted or unsubstituted C₁-C₈ alkyl and substituted or unsubstituted C₃-C₆ cycloalkyl, wherein the C₁-C₈ alkyl or C₃-C₆ cycloalkyl comprises at least one fluorine atom substituent;
n is selected from 1, 2, or 3;
the "substituted" means that one, two, three, or four hydrogen atoms in a group are each independently replaced by a substituent selected from the group consisting of deuterium, halogen, halogen-substituted or unsubstituted C₁₋₆ alkyl, halogen-substituted or unsubstituted C₃₋₆ cycloalkyl, deuterium-substituted or unsubstituted C₁₋₆ alkyl, and deuterium-substituted or unsubstituted C₃₋₆ cycloalkyl.

2. The compound according to claim 1, wherein X₁ is hydrogen.

3. The compound according to claim 1 or 2, wherein X₂ is selected from C₁-C₈ alkyl substituted with 1, 2, or 3 fluorine;
preferably, X₂ is selected from -CH₂-CF₃, -CH(CH₃)-CF₃, or -C(CH₃)₂-CF₃.

4. A compound of Formula (II) or Formula (III), and a pharmaceutically acceptable salt and a stereoisomer thereof: 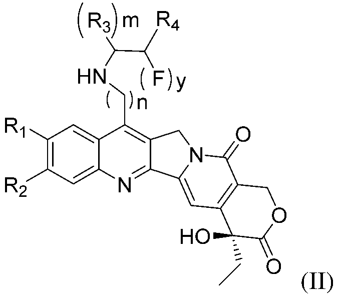 wherein,
R₁ and R₂ are each independently selected from hydrogen, fluorine, and methyl, or
R₁ and R₂ together with the carbon atoms to which they are attached form 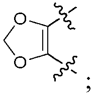
R₃ and R₄ are each independently selected from hydrogen, fluorine, and substituted or unsubstituted C₁-C₆ alkyl, or
R₃ and R₄ together with the carbon atoms to which they are attached form C₃-C₆ cycloalkyl;
n is selected from 1, 2, or 3;
m is selected from 1 or 2, and y is selected from 1 or 2;
the "substituted" means that one, two, three, or four hydrogen atoms in a group are each independently replaced by a substituent selected from the group consisting of deuterium, halogen, halogen-substituted or unsubstituted C₁₋₆ alkyl, halogen-substituted or unsubstituted C₃₋₆ cycloalkyl, deuterium-substituted or unsubstituted C₁₋₆ alkyl, and deuterium-substituted or unsubstituted C₃₋₆ cycloalkyl; 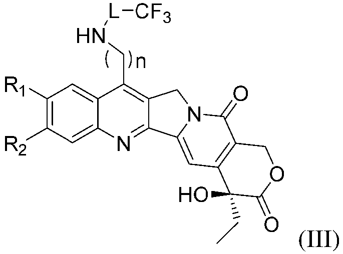
in Formula (III), R₁, R₂, and n are each the same as defined for the compound of Formula (I);
L is selected from substituted or unsubstituted C₁-C₆ alkyl;
the "substituted" means that one, two, three, or four hydrogen atoms in a group are each independently replaced by a substituent selected from the group consisting of deuterium, halogen, and halogen-substituted or unsubstituted C₁₋₆ alkyl.

5. The compound according to claim 4, wherein the compound is selected from compounds represented by Formula (II-1), Formula (II-2), Formula (II-3), or Formula (II-4): 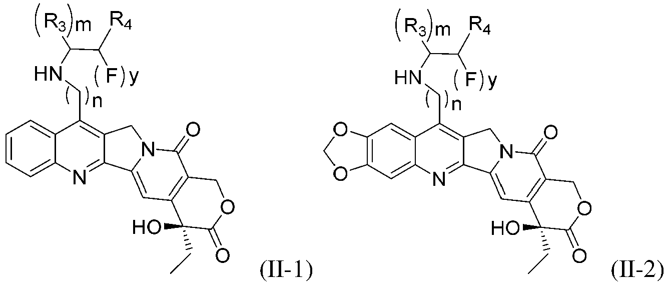 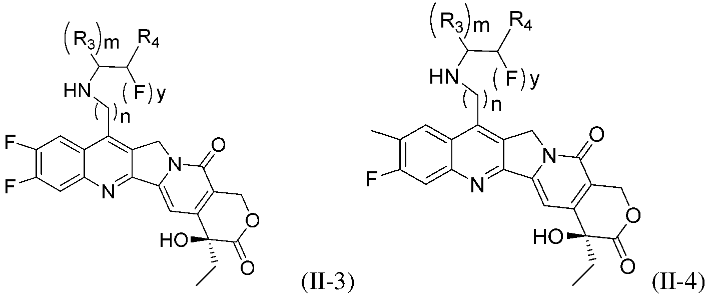 in the formulas, R₃, R₄, n, m and y are each the same as defined for the compound of Formula (II).

6. The compound according to claim 4 or 5, wherein R₃ is selected from hydrogen and substituted or unsubstituted C₁-C₆ alkyl, preferably is hydrogen or methyl;
preferably, R₄ is selected from hydrogen or fluorine, more preferably is fluorine;
preferably, y is 2.

7. The compound as described below, and a pharmaceutically acceptable salt and stereoisomer thereof:

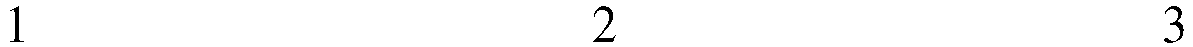

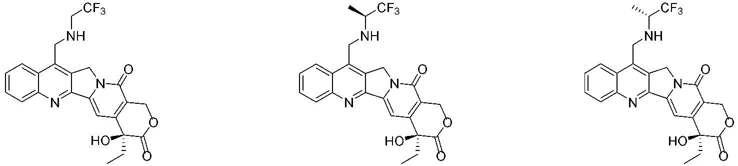

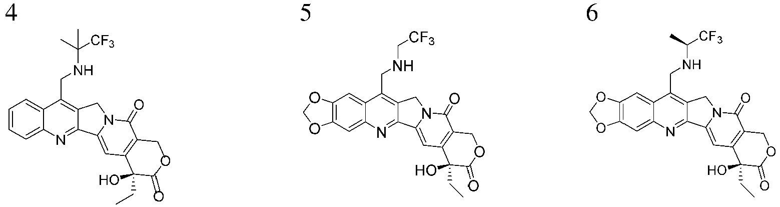

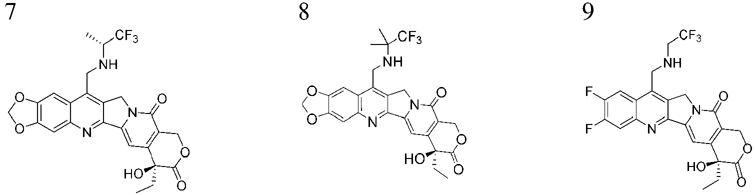

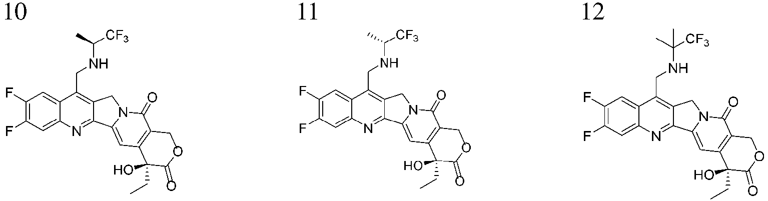

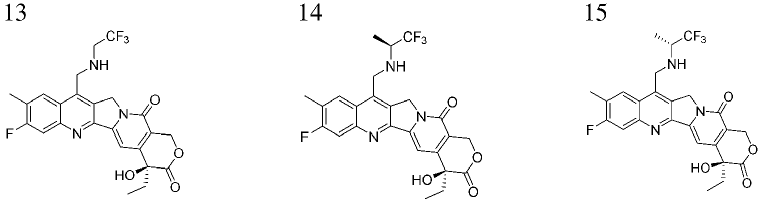

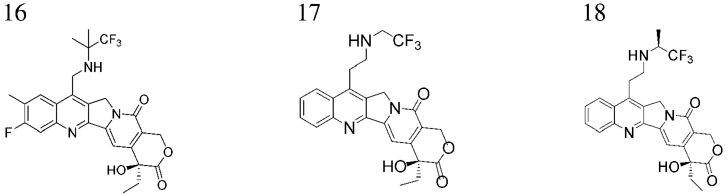

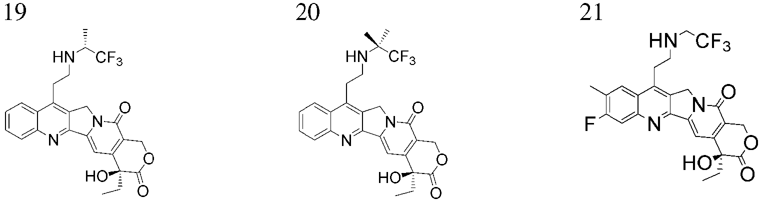

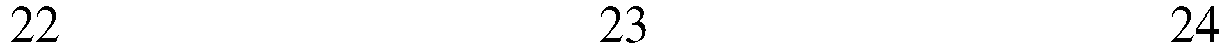

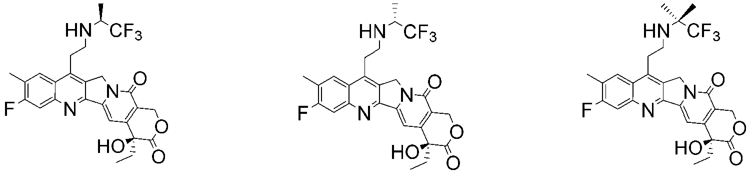

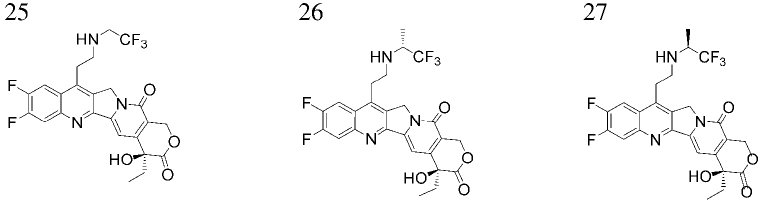

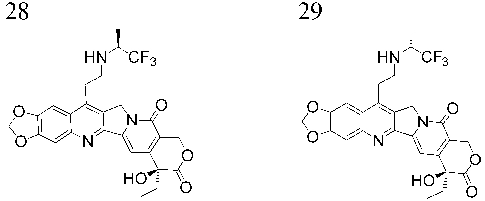

8. A method for preparing the compound according to any one of claims 1 to 7, comprising the step of subjecting a 7-formylcamptothecin derivative and a corresponding fluorine-substituted amine to reductive amination reaction;
wherein, the 7-formylcamptothecin derivative is selected from any one of 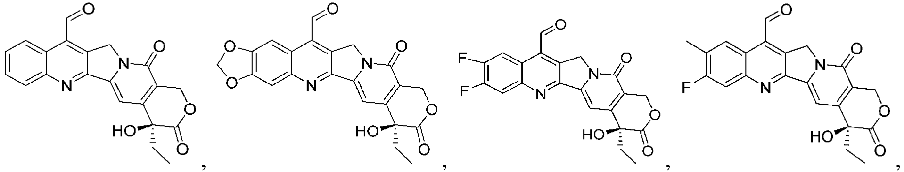 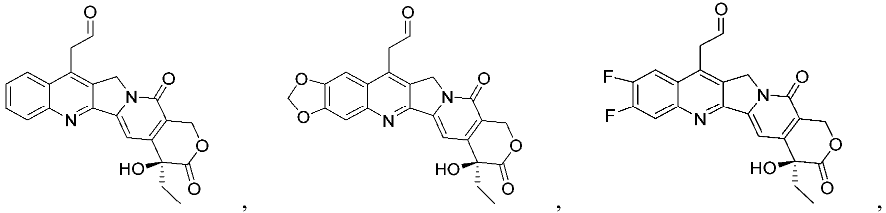 and 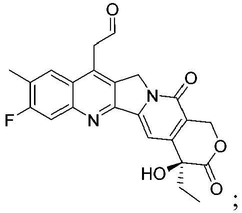
the corresponding fluorine-substituted amine is selected from at least one of the following compounds: 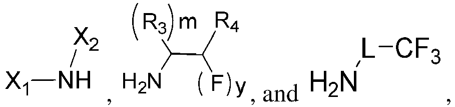
wherein, X₁, X₂, R₃, R₄, m, n, y, and L are each the same as defined above; and
preferably, the corresponding fluorine-substituted amine is selected from

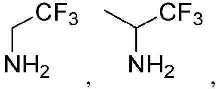

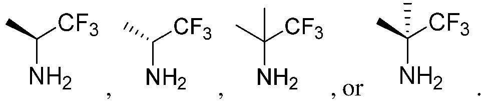

9. A pharmaceutical composition, comprising the compound or the pharmaceutically acceptable salt or stereoisomer thereof according to any one of claims 1 to 8, and a pharmaceutically acceptable adjuvant.

10. Use of the compound or the pharmaceutically acceptable salt or stereoisomer thereof according to any one of claims 1 to 8 or the pharmaceutical composition according to claim 9 in the manufacture of a medicament for treating a cancer;
preferably, the cancer is selected from one or more of esophageal cancer, gastric cancer, lung cancer, and breast cancer.

11. A method for treating a cancer, comprising administering the compound or the pharmaceutically acceptable salt or stereoisomer thereof according to any one of claims 1 to 8 or the pharmaceutical composition according to claim 9 to a subject;
preferably, the cancer is selected from one or more of esophageal cancer, gastric cancer, lung cancer, and breast cancer.
